# EUROPÄISCHE PATENTSCHRIFT

(11) **EP 0 569 795 B1**
(45) Veröffentlichungstag und Bekanntmachung des Hinweises auf die Patenterteilung: **12.04.1995**
(21) Anmeldenummer: 93106988.4
(22) Anmeldetag: 29.04.1993
(51) Int. Cl.: C07D 401/12, A61K 31/44, C07D 215/227

(54) **Sulfonylbenzyl-substituierte Benzo- und Pyridopyridone**
Sulphonylbenzyl substituted benzo- and pyridopyridones
Benzo- et pyridopyridones substitués par sulfonylebenzyle

(30) Priorität: 12.05.1992 DE 4215587
(43) Veröffentlichungstag der Anmeldung: 18.11.1993
(73) Patentinhaber: BAYER AG, 51368 Leverkusen (DE)
(72) Erfinder: Dressel, Jürgen, Dr., W-5600 Wuppertal 1 (DE); Fey, Peter, Dr., W-5600 Wuppertal 1 (DE); Hanko, Rudolf H., Dr., W-4000 Düsseldorf 1 (DE); Hübsch, Walter, Dr., W-5600 Wuppertal 1 (DE); Krämer, Thomas, Dr., W-5600 Wuppertal 1 (DE); Müller, Ulrich E., Dr., W-5600 Wuppertal 1 (DE); Müller-Gliemann, Matthias, Dr., W-5650 Solingen-Ohligs (DE); Beuck, Martin, Dr., W-4006 Erkrath 2 (DE); Kazda, Stanislav, Prof. Dr., W-5600 Wuppertal 1 (DE); Wohlfeil, Stefan, Dr., W-4010 Hilden (DE); Knorr, Andreas, Dr., W-4006 Erkrath 2 (DE); Stasch, Johannes-Peter, Dr., W-5600 Wuppertal 1 (DE)

(56) Entgegenhaltungen:
- EP-A- 0 412 848
- EP-A- 0 456 442
- EP-A- 0 475 206
- EP-A- 0 487 252
- EP-A- 0 512 676
- EP-A- 0 534 706
- JP-A- 3 178 966
- JP-A-61 045 820
- US-A- 3 914 236

## Beschreibung

Die vorliegende Erfindung betrifft Sulfonylbenzyl-substituierte Benzo- und Pyridopyridone, ein Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksenkenden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckehöhung.

Darüberhinaus besitzt Angiotensin II die Eigenschalt, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

In JP-B 3 178 966 sowie in JP-B 61 045 820 werden am Ring-Stickstoff unsubstituierte Benzo-pyridone beschrieben.

In US 3 914 236 werden Imidazolderivate offenbart, die am Stickstoff duch einen Isochinolinrest substituiert sind. Die Verbindungen eignen sich zur Behandlung von Diabetes.

Die vorliegende Erfindung betrifft Sulfonylbenzyl-substituierte Benzo- und Pyridopyridone der allgemeinen Formel (I) 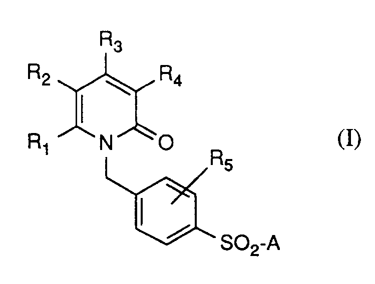
in welcher
- R¹und R²: gleich oder verschieden sind und für Wasserstoff, Cyano oder
für geradkettiges oder verzweigtes Alkyl, Alkenyl oder Alkinyl mit jeweils bis zu 8 Kohlenstoffatomen stehen, die gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen oder Phenyl substituiert sind, oder
für Cycloalkyl mit 3 bis 6 Kohlenstoffatomen stehen
für geradkettiges oder verzweigtes Acyl oder Alkoxycarbonyl mit jeweils bis zu 8 Kohlenstoffatomen, Benzyloxycarbonyl oder Carboxy stehen, oder
für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder für die Gruppe der Formel -CO-NR⁶R⁷, B-R⁸ oder -NR⁹R¹⁰ stehen,
worin
- R⁶ und R⁷: gleich oder verschieden sind und Wasserstoff, Phenyl, geradkettiges oder verzweigtes Alkyl mit bis zu 6 Kohlenstoffatomen oder Benzyl bedeuten,
- B: ein Sauerstoff- oder Schwefelatom bedeutet,
- R⁸: geradkettiges oder verzweigtes Alkyl mit bis zu 8 Kohlenstoffatomen bedeutet,
- R⁹ und R¹⁰: gleich oder verschieden sind und die oben angegebene Bedeutung von R⁶ und R⁷ haben, oder
- R⁹ oder R¹⁰: die -SO₂R¹¹-Gruppe bedeutet,
worin
- R¹¹: geradkettiges oder verzweigtes Alkyl mit bis zu 6 Kohlenstoffatomen, Benzyl oder Phenyl bedeutet, die gegebenenfalls durch Methyl substituiert sind,
- R³ und R⁴: unter Einbezug der Doppelbindung einen Phenyl- oder Pyridylring ausbilden, der gegebenenfalls bis zu 3-fach gleich oder verschieden durch Hydroxy, Formyl, Carboxy, Halogen, geradkettiges oder verzweigtes Acyl oder Alkoxycarbonyl mit jeweils bis zu 8 Kohlenstoffatomen, geradkettiges oder verzweigtes Perfluoralkyl mit bis zu 6 Kohlenstoffatomen oder durch geradkettiges oder verzweigtes Alkyl mit bis zu 8 Kohlenstoffatomen substituiert ist, das seinerseits durch Hydroxy oder geradkettiges oder verzweigtes Alkoxy mit bis zu 6 Kohlenstoffatomen substituiert sein kann, oder durch die Gruppe der Formel -CONR⁶R⁷ substituiert ist,
worin
- R⁶ und R⁷: die oben angegebene Bedeutung haben,
- R⁵: für Wasserstoff, Nitro, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder
für eine Gruppe der Formel -OR¹², -NR¹³R¹⁴ oder -NR¹³-CO-R¹⁵ steht,
worin
- R¹²: Wasserstoff, geradkettiges oder verzweigtes Alkyl mit bis zu 8 Kohlenstoffatomen oder Phenyl bedeutet,
- R¹³ und R¹⁴: gleich oder verschieden sind und die oben angegebene Bedeutung von R⁶ und R⁷ haben
und
- R¹⁵: geradkettiges oder verzweigtes Alkyl mit bis zu 6 Kohlenstoffatomen oder Phenyl bedeutet
- A: für einen über das Stickstoffatom gebundenen 3- bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel
-NR^{9'}R^{10'}, -SO₃H , 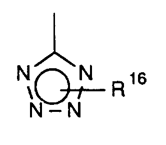 oder -CO-R¹⁷ substituiert ist,
worin
- R^{9'} und R^{10'}: die oben angegebene Bedeutung von R⁹ und R¹⁰ haben und mit dieser gleich oder verschieden sind,
- R¹⁶: Wasserstoff, geradkettiges oder verzweigtes Alkyl mit bis zu 6 Kohlenstoffatomen oder Triphenylmethyl bedeutet
und
- R¹⁷: Hydroxy, geradkettiges oder verzweigtes Alkoxy mit bis zu 8 Kohlenstoffatomen, Phenoxy oder eine Gruppe der Formel - NR¹⁸R¹⁹ bedeutet,
worin
- R¹⁸ und R¹⁹: gleich oder verschieden sind und Wasserstoff oder geradkettiges oder verzweigtes Alkyl mit bis zu 6 Kohlenstoffatomen oder Phenyl bedeuten,
und deren Salze.

Die erfindungsgemäßen Sulfonylbenzyl-substituierten Benzo- und Pyridopyridone können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Sulfonylbenzyl-substituierten Benzo- und Pyridopyridone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomere oder als Diastereomere, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Ein über das Stickstoffatom gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidinyl. Bevorzugt sind 5- und 6-gliedrige Ringe mit einem Sauerstoff- und/oder bis zu 2-Stickstoffatomen, wie beispielsweise Piperidyl, Morpholinyl oder Pyrrolidinyl. Besonders bevorzugt sind Piperidyl und Pyrrolidinyl.

Bevorzugt sind Verbindungen der allgemeinen Formel (I)
in welcher
- R¹und R²: gleich oder verschieden sind und für Wasserstoff, Cyano, oder
für geradkettiges oder verzweigtes Alkyl, Alkenyl oder Alkinyl mit jeweils bis zu 6 Kohlenstoffatomen stehen, die gegebenenfalls durch Cyclopropyl, Cyclopentyl, Cyclohexyl, Hydroxy oder durch geradkettiges oder verzweigtes Alkoxy mit bis zu 4 Kohlenstoffatomen oder Phenyl substituiert sind, oder
für Cyclopropyl, Cyclopentyl oder Cyclohexyl Phenyl stehen,
für geradkettiges oder verzweigtes Acyl oder Alkoxycarbonyl mit jeweils bis zu 6 Kohlenstoffatomen, Benzyloxycarbonyl oder Carboxy stehen, oder
für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder
für eine Gruppe der Formel -CO-NR⁶R⁷, B-R⁸ oder -NR⁹R¹⁰ stehen,
worin
- R⁶ und R⁷: gleich oder verschieden sind und Wasserstoff, Phenyl, geradkettiges oder verzweigtes Alkyl mit bis zu 4 Kohlenstoffatomen oder Benzyl bedeuten,
- B: ein Sauerstoff- oder Schwefelatom bedeutet,
- R⁸: geradkettiges oder verzweigtes Alkyl mit bis zu 6 Kohlenstoffatomen bedeutet,
- R⁹ und R¹⁰: gleich oder verschieden sind und die oben angegebene Bedeutung von R⁶ und R⁷ haben oder R⁹ oder R¹⁰ die -SO₂R¹¹-Gruppe bedeutet,
worin
- R¹¹: geradkettiges oder verzweigtes Alkyl mit bis zu 4 Kohlenstoffatomen,Phenyl oder Tolyl bedeutet,
- R³ und R⁴: gemeinsam unter Einbezug der Doppelbindung einen Phenyl- oder Pyridylring ausbilden, der gegebenenfalls bis zu 2-fach gleich oder verschieden durch Hydroxy, Formyl, Carboxy, Fluor, Chlor, Brom, geradkettiges oder verzweigtes Acyl oder Alkoxycarbonyl mit jeweils bis zu 6 Kohlenstoffatomen, geradkettiges oder verzweigtes Perfluoralkyl mit bis zu 4 Kohlenstoffatomen oder durch geradkettiges oder verzweigtes Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann, oder durch die Gruppe der Formel -CONR⁶R⁷ substituiert ist,
worin
- R⁶ und R⁷: die oben angegebene Bedeutung haben,
- R⁵: für Wasserstoff, Nitro, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht, oder
für eine Gruppe der Formel -OR¹², -NR¹³R¹⁴ oder -NR¹³-CO-R¹⁵ steht,
worin
- R¹²: Wasserstoff oder geradkettiges oder verzweigtes Alkyl mit bis zu 6 Kohlenstoffatomen bedeutet,
- R¹³ und R¹⁴: gleich oder verschieden sind und die oben angegebene Bedeutung von R⁶ und R⁷ haben
und
- R¹⁵: geradkettiges oder verzweigtes Alkyl mit bis zu 6 Kohlenstoffatomen oder Phenyl bedeutet
- A: für über das Stickstoffatom gebundenes Piperidyl, Pyrrolidinyl oder Morpholinyl steht, die gegebenenfalls durch einen Rest der Formel
NR^{9'}R^{10'}, -SO₃H , 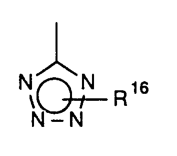 oder -CO-R¹⁷ substituiert sind,
worin
- R^{9'} und R^{10'}: die oben angegebene Bedeutung von R⁹ und R¹⁰ haben und mit dieser gleich oder verschieden sind,
- R¹⁶: Wasserstoff, geradkettiges oder verzweigtes Alkyl mit bis zu 4 Kohlenstoffatomen oder Triphenylmethyl bedeutet
und
- R¹⁷: Hydroxy, geradkettiges oder verzweigtes Alkoxy mit bis zu 6 Kohlenstoffatomen, Phenoxy oder eine Gruppe der Formel -NR¹⁸R¹⁹ bedeutet,
worin
- R¹⁸ und R¹⁹: gleich oder verschieden sind und Wasserstoff oder geradkettiges oder verzweigtes Alkyl mit bis zu 4 Kohlenstoffatomen bedeuten,
und deren Salze.

Besonders bevorzugt sind Verbindungen der allgemeinen Formel (I),
in welcher
- R¹ und R²: gleich oder verschieden sind und für Wasserstoff, Cyano oder
für geradkettiges oder verzweigtes Alkyl mit bis zu 4 Kohlenstoffatomen stehen, das gegebenenfalls durch Cyclopropyl substituiert ist oder
für Cyclopropyl oder Phenyl stehen, oder
für geradkettiges oder verzweigtes Acyl oder Alkoxycarbonyl mit jeweils bis zu 4 Kohlenstoffatomen, Benzyloxycarbonyl oder Carboxy stehen, oder
für eine Gruppe der Formel -CO-NR⁶R⁷, B-R⁸ oder NR⁹R¹⁰ stehen,
worin
- R⁶ und R⁷: gleich oder verschieden sind und Wasserstoff, Phenyl, Ethyl oder Benzyl bedeuten,
- B: ein Sauerstoff- oder Schwefelatom bedeutet,
- R⁸: geradkettiges oder verzweigtes Alkyl mit bis zu 4 Kohlenstoffatomen bedeutet,
- R⁹ und R¹⁰: gleich oder verschieden sind und die oben angegebene Bedeutung von R⁶ und R⁷ haben oder R⁹ oder R¹⁰ die -SO₂R¹¹-Gruppe bedeutet,
worin
- R¹¹: Methyl, Phenyl oder Tolyl bedeutet,
- R³ und R⁴: gemeinsam unter Einbezug der Doppelbindung einen ankondensierten Phenyl- oder Pyridylring ausbilden, der gegebenenfalls bis zu 2-fach gleich oder verschieden durch Hydroxy, Carboxy, Fluor, Chlor, geradkettiges oder verzweigtes Acyl oder Alkoxycarbonyl mit jeweils bis zu 4 Kohlenstoffatomen oder geradkettiges oder verzweigtes Perfluoralkyl mit bis zu 3 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann,
oder
durch die Gruppe der Formel -CO-NR⁶R⁷ substituiert ist,
worin
- R⁶und R⁷: die oben angegebene Bedeutung haben,
- R⁵: für Wasserstoff, Nitro, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht, oder
für eine Gruppe der Formel -OR¹², -NR¹³R¹⁴ oder -NR¹³-CO-R¹⁵ steht,
worin
- R¹²: Wasserstoff oder geradkettiges oder verzweigtes Alkyl mit bis zu 4 Kohlenstoffatomen bedeutet,
- R¹³ und R¹⁴: gleich oder verschieden sind und die oben angegebene Bedeutung von R⁶ und R⁷ haben
und
- R¹⁵: geradkettiges oder verzweigtes Alkyl mit bis zu 6 Kohlenstoffatomen oder Phenyl bedeutet
- A: für über das Stickstoffatom gebundenes Piperidyl oder Pyrrolidinyl steht, die gegebenenfalls durch einen Rest der Formel
-NR^{9'}R^{10'}, -SO₃H , 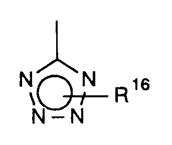 oder -CO-R¹⁷ substituiert sind,
worin
- R^{9'} und R^{10'}: die oben angegebene Bedeutung von R⁹ und R¹⁰ haben und mit dieser gleich oder verschieden sind,
- R¹⁶: Wasserstoff, Methyl, Ethyl oder Triphenylmethyl bedeutet
und
- R¹⁷: Hydroxy, geradkettiges oder verzweigtes Alkoxy mit bis zu 4 Kohlenstoffatomen, Phenoxy oder eine Gruppe der Formel -NR¹⁸R¹⁹ bedeutet,
worin
- R¹⁸ und R¹⁹: gleich oder verschieden sind und Wasserstoff oder geradkettiges oder verzweigtes Alkyl mit bis zu 3 Kohlenstoffatomen bedeuten
und deren Salze.

Außerdem wurde ein Verfahren zur Herstellung der erfindungsgemäßen Verbindungen der allgemeinen Formel (I) gefunden, dadurch gekennzeichnet, daß man
Pyridone der allgemeinen Formel (II) 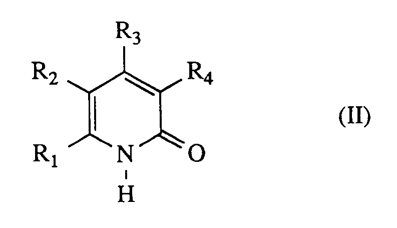
in welcher
- R¹, R², R³ und R⁴: die oben angegebene Bedeutung haben,
mit Verbindungen der allgemeinen Formel (III) 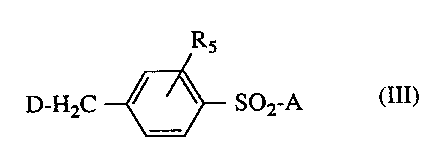
in welcher
- R⁵ und A: die oben angegebene Bedeutung haben
und
- D: für Halogen, vorzugsweise für Brom steht,
in organischen Lösemitteln und in Anwesenheit einer Base und gegebenenfalls eines Katalysators umsetzt,
und im Fall, daß R¹⁶ nicht für Wasserstoff steht, eine Alkylierung anschließt,
und im Fall der Säuren (R¹⁷ = OH) die entsprechenden Ester verseift,
und im Fall der Ester oder Amide, gegebenenfalls über eine aktivierte Carbonsäurestufe, eine Veresterung bzw. Amidierung anschließt,
und sowohl die Substituenten R¹, R² und R⁵ als auch die Substituenten des Phenyl- und Pyridylrings (R³/R⁴) nach üblichen Methoden variiert

Das erfindungsgemäße Verfahren kann durch folgendes Formelschema beispielhaft erläutert werden: 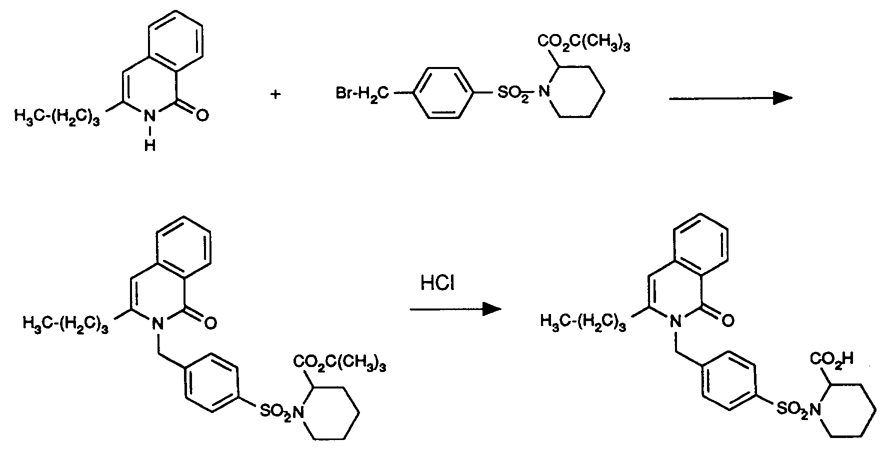
Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, 1,2-Dimethoxyethan oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Tetrahydrofuran und 1,2-Dimethoxyethan.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid, Kaliumhydroxid oder Lithiumhydroxid, Bariumhydroxid, Alkali- oder Erdalkalicarbonate wie Natriumcarbonat, Kaliumcarbonat, Calciumcarbonat oder Caesiumcarbonat, oder Alkali- oder Erdalkalialkoholate wie Natrium- oder Kaliummethanolat oder Kalium-tert.-butylat, oder Lithiumdiisopropylamid (LDA), oder organische Amine (Trialkyl-(C₁-C₆)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat und Caesiumcarbonat.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100°C bis +100°C, bevorzugt von 0°C bis 40°C durchgeführt

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0°C bis 150°C, vorzugsweise von 20°C bis 100°C und Normaldruck.

Als Katalysatoren eignen sich Kalium- oder Natriumiodid, bevorzugt Natriumiodid.

Die Alkylierung erfolgt im allgemeinen mit Alkylierungsmitteln wie beispielsweise (C₁-C₆)-Alkylhalogeniden, Sulfonsäurestern oder substituierten oder unsubstituierten (C₁-C₆)-Dialkyl- oder (C₁-C₆)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid oder Dimethoxyethan in einem Temperaturbereich von 0°C bis +70°C, vorzugsweise von 0°C bis +30°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid, Kaliumhydroxid oder Lithiumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen. Bevorzugt sind Tetrahydrofuran und Methanol.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +80°C, vorzugsweise von -10°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Frerman et al., J. Biol. Chem. 225, 507 (1982) und N.B. Benoton, K. Kluroda, Inc Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Pyridone der allgemeinen Formel (II) sind teilweise neu, solche Verbindungen sind z.B. schon aus die auf Seite Ia zitierten Patentschriften bekannt und können hergestellt werden,
[A] im Fall, daß
   - R³ und R⁴: einen Phenylring ausbilden, indem man Verbindungen der allgemeinen Formel (IV) 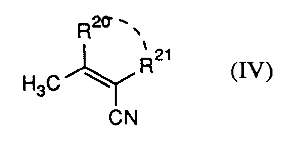
   in welcher
   - R²⁰ und R²¹: einen gegebenenfalls substituierten Phenylring bilden,
   zunächst durch Umsetzungen mit Verbindungen der allgemeinen Formel (V)

   R^{1'}-CO₂-E (V)

   in welcher
   - R^{1'}: für einen Alkyl-, Alkenyl- oder Alkinyl-Rest steht,
   und
   - E: für C₁-C₄-Alkyl, vorzugsweise für Methyl steht,
   unter Schutzgasatmosphäre, in einem der oben aufgeführten Lösemittel und in Anwesenheit einer der dort ebenfalls aufgeführten Basen, und/oder Katalysatoren
   vorzugsweise in Ammoniak mit Kaliumamid in die Verbindungen der allgemeinen Formel (VI) 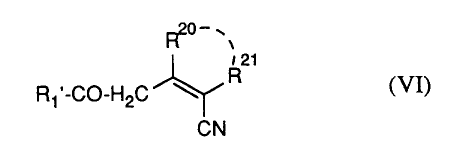 in welcher
   - R^{1'}, R²⁰ und R²¹: die oben angegebene Bedeutung haben,
   überführt, anschließend mit Säuren in Alkoholen, vorzugsweise mit Schwefelsäure in Ethanol, cyclisiert,
   und im Fall, daß R¹ nicht für einen Alkyl-, Alkenyl- oder Alkinylrest steht, den Substituenten R^{1'} nach üblichen Methoden derivatisiert,
[B] im Fall, daß
   - R³ und R⁴: gemeinsam einen Pyridylring ausbilden, indem man beispielsweise Pyridine der allgemeinen Formel (VII) 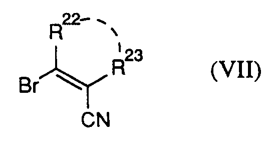
   in welcher
   - R²² und R²³: einen gegebenenfalls substituierten Pyridylring bilden,
   zunächst durch Umsetzung mit Verbindungen der allgemeinen Formel (VIII)

   CH≡C-R¹ (VIII)

   in welcher
   - R¹: die oben angegebene Bedeutung hat,
   im Autoklaven unter Schutzgasatmosphäre in Anwesenheit von Katalysatoren/Hilfsstoffen, vorzugsweise im System Bis-(triphenylphosphin)-Palladium(II)chlorid / Kupfer(I)iodid in die Verbindungen der allgemeinen Formel (IX) 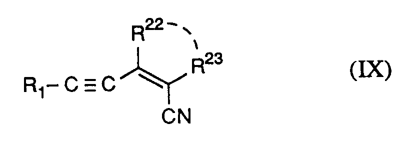 in welcher
   - R¹, R²² und R²³: die oben angegebene Bedeutung haben,
   überführt und anschließend entweder direkt wie unter [A] beschrieben cyclisiert oder zunächst über die Stufe der allgemeinen Formel (X) 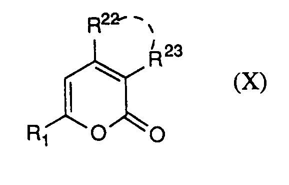 in welcher
   - R¹, R²² und R²³: die oben angegebene Bedeutung haben,
   cyclisiert und anschließend mit Ammoniak umsetzt,
   und im Fall, daß R² nicht für Wasserstoff steht, nach üblichen Methoden derivatisiert
   und ebenso die unter R³/R⁴ aufgeführten Phenyl- als auch die Pyridylsubstituenten gegebenfalls variiert.

Die Katalysatoren/Hilfsstoffe werden im allgemeinen in einer Menge von 0,001 mol bis 0,5 mol, bevorzugt von 0,01 mol bis 0,3 mol, jeweils bezogen auf 1 mol der Verbindungen der allgemeinen Formeln (IV) und (VII) eingesetzt.

Die Basen werden im allgemeinen in einer Menge von 1 mol bis 5 mol, bevorzugt von 1 mol bis 3 mol, jeweils bezogen auf 1 mol der Verbindungen der allgemeinen Formel (IV) eingesetzt.

Die Reaktionstemperaturen für die einzelnen Schritte liegen in einem Bereich von 0°C bis 180°C, vorzugsweise von 20°C bis 150°C.

In Abhängigkeit der einzelnen Reaktionsschritte kann sowohl bei Normaldruck als auch bei erhöhtem Druck, beispielsweise 0,5 bis 5 bar, und gegebenenfalls unter Schutzgasatmosphäre gearbeitet werden.

Die Verbindungen der allgemeinen Formeln (IV), (V) und (VI) sind größtenteils bekannt oder können nach üblichen Methoden hergestellt werden (vgl. z.B. J.Org. Chem. 1966, 31, 3807).

Die Verbindungen der allgemeinen Formel (VII) sind teilweise neu und können beispielsweise hergestellt werden, indem man zunächst die entsprechenden 3-Brom substituierten Pyridine durch Umsetzung mit Wasserstoffperoxid in Essigsäure zu den jeweiligen Pyridin-N-oxiden umsetzt und in einem zweiten Schritt die Cyano-Gruppe nach üblichen Methoden, beispielsweise mit Trimethylsilylcyanid in Acetonitril und in Anwesenheit von Triethylamin, in einem Temperaturbereich von 20°C bis 120°C, vorzugsweise von 60°C bis 100°C, einführt (vgl. hierzu J.Org. Chem. 1958, 23, 1616, Chem. Pharm. Bull. 1985, 35, 565).

Die Verbindungen der allgemeinen Formel (VIII) sind an sich bekannt oder können nach üblichen Methoden hergestellt werden.

Die Verbindungen der allgemeinen Formeln (IX) und (X) sind teilweise bekannt oder neu und können beispielsweise wie oben beschrieben oder nach publizierten Methoden hergestellt werden (vgl. z.B. Chem. Pharm. Bull, 34(7) 2760-5; 33(2), 626-33; Heterocycles 32(5), 1013-16 Indian J.Chem., Sect. B, 20B(5), 376-9; Chem. Pharm. Bull. 1988, 36, 1890).

Die Verbindungen der allgemeinen Formel (III) sind neu und können hergestellt werden, indem man substituierte Benzylsulfonsäurechloride der allgemeinen Formel (XI) 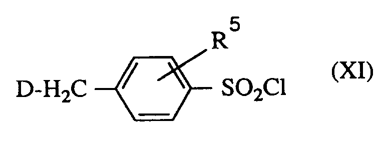
in welcher
- R⁵: die oben angegebene Bedeutung hat
und
- D: für Halogen, vorzugsweise für Brom steht,
mit Verbindungen der allgemeinen Formel (XII)

H-A (XII)

in welcher
- A: die oben angegebene Bedeutung hat,
in einem der oben angegebenen Lösemittel und Basen, vorzugsweise in Dichlormethan mit Triethylamin umsetzt.

Die Umsetzung wird im allgemeinen in einem Temperaturbereich von 0°C bis +100°C, bevorzugt von +20°C bis +80°C durchgeführt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Bei der Durchführung der Umsetzung wird die Base im allgemeinen in einer Menge von 1 bis 3 mol, bevorzugt von 1 bis 1,5 mol bezogen auf 1 mol der Verbindungen der allgemeinen Formel (IV), eingesetzt. Besonders bevorzugt verwendet man molare Mengen der Reaktanden.

Die Umsetzung erfolgt im allgemeinen in einem Temperaturbereich von -10°C bis +40°C, vorzugsweise von -10°C bis 0°C und unter Normaldruck.

Die Verbindungen der allgemeinen Formeln (XI) und (XII) sind bekannt oder können nach üblicher Methode hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an A II-Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen Applikationsvolumen pro Einzelyabe = 100 »l): | | |
|---|---|---|
| KCl | 22,7;32,7;42,7;52,7 | mmol/l |
| 1 Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻7;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

**Tabelle A**

| Hemmung der Gefäßkontraktion an isolierten Aortenringen von Kaninchen in vitro | |
|---|---|
| Beispiel 2 | IC₅₀ = 660 nM |

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 »g/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht. Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark und Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 »g), ³H-Angiotensin II (3-5 nM), Test-Pufferlösung (50 mM Tris, pH 7,2, 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten oder Schweinen durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium mit Serumzusatz, 2 mmol L-Glutamin und 15 mmol HEPES, pH 7,4 in 5% CO₂ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzc Nach 16 - 20 Stunden wird 1 »Ci ³H-Thymidin zugefügt und nach weiteren 4 Stunden der Einbau dieser Substanz in die TCA-präzipitierbare DNA der Zellen bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### 2-(2-Oxo-hexyl)-benznitril

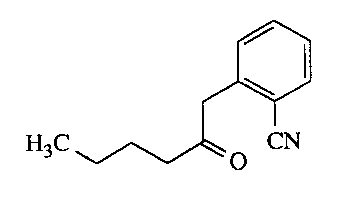

Unter Argon wird Kalium (3,8 g, 0,10 mol) in Ammoniak (150 ml) gelöst, mit einer Spatelspitze Eisen-III-nitrat versetzt und 15 min unter Rückfluß gerührt. Eine Lösung von 2-Toluolnitril (12 ml; 0,10 mol) in Ether (25 ml) wird zugetropft, des weiteren wird nach 10 min eine Lösung von Valeriansäuremethylester (6,6 ml; 0,050 mol) in Ether (25 ml) zugefügt. Nach einer Stunde werden Ammoniumchlorid (6,1 g, 0,12 mol) und Ether (25 ml) zugegeben, und über Nacht der Ammoniak abgedampft. Die Suspension wird kurz erhitzt, mit 6 N-Salzsäure sauer gestellt und mit Methylenchlorid extrahiert. Trocknen der organischen Phase über Natriumsulfat, Einengen und Kieselgelchromatographie (Hexan : Essigester = 5:1) liefern 3,1 g eines gelben Öls (31% der Theorie).
R_{f} = 0,52 (Hexan : Ethylacetat = 3 : 1).

### Beispiel II

### 3-Butyl-isochinolin-1(2H)-on

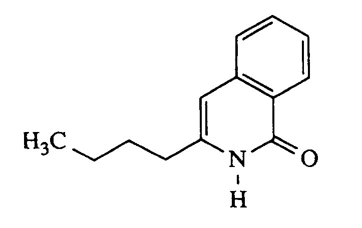

Unter Eiskühlung wird zu einer Lösung von Beispiel I (3,1 g; 15 mmol) in Ethanol/Wasser (19:1; 600 ml) konzentrierte Schwefelsäure (60 ml) gegeben. Nach 7 h Erhitzen zum Rückfluß wird die Reaktionslösung auf Eis gegossen und eingeengt. Absaugen und Umkristallisation des ausgefallenen Produkts aus Hexan ergeben 1,8 g eines weißen Feststoffs (57% der Theorie).
Fp.: 137°C
R_{f} = 0,28 (Hexan : Essigester = 3:1)

### Beispiel III

### 2-(2-Oxo-butyl)-benznitril

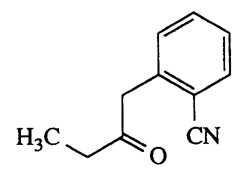

Analog zur Beispiel I werden durch Acylierung von 2-Tolylnitril (1 ml; 0,10 mol) mit Propionsäuremethylester (4,8 ml; 50 mmol) 3,1 g eines gelben Öls erhalten (36% der Theorie).
R_{f} = 0,46 (Hexan : Essigester = 3:1)

### Beispiel IV

### 3-Ethyl-isochinolin-1(2H)-on

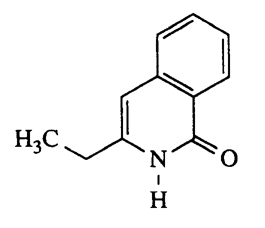

Analog Beispiel II werden aus Beispiel III (3,1 g; 18 mmol) 1,6 g eines Feststoffs erhalten (52% der Theorie).
Fp.: 136°C
R_{f} = 0,13 (Hexan : Essigester = 3:1)

### Beispiel V

### 2-(Benzoylmethyl)-benznitril

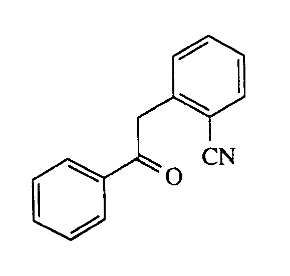

Analog zur Beispiel I werden durch Acylierung von 2-Tolylnitril (12 ml; 0,10 mol) mit Benzoesäuremethylester (6,3 ml; 50 mmol) 3,1 g eines weißen Feststoffs erhalten (50% der Theorie).
Fp.: 109°C
R_{f} = 0,42 (Hexan : Essigester = 3:1)

### Beispiel VI

### 3-Phenyl-isochinolin-1(2H)-on

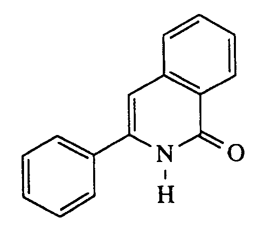

Analog Beispiel II werden aus Beispiel V (4,3 g; 19 mmol) 2,0 g eines Feststoffs erhalten (46% der Theorie).
Fp.: 105°C
R_{f} = 0,15 (Hexan : Essigester = 3:1)

### Beispiel VII

### 3-Brompyridin-N-oxid

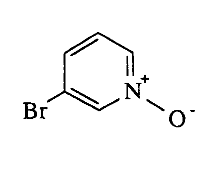

Eine Lösung von 3-Brompyridin (3 ml; 0,32 mol) in Eisessig (250 ml) wird mit Wasserstoffperoxid: H₂O (30:70; 50 ml) versetzt und bei 100°C gerührt. Nach 3 h und 19 h wird weiteres Wasserstoffperoxid:H₂O (30:70; je 25 ml) zugegeben und noch 4 h auf 100°C erhitzt. Die Reaktionslösung wird auf ein Drittel des Volumens eingeengt, mit Wasser wieder aufgefüllt und vollständig eingeengt. Der Rückstand wird in Methylenchlorid gelöst und mit Natriumcarbonatlösung gewaschen. Sättigen der wäßrigen Phase mit Kochsalz, Extraktion mit Methylenchlorid sowie Trocknen und Einengen der vereinigten organischen Phasen liefern 43 g eines Öls (77% der Theorie).
R_{f} = 0,37 (Methylenchlorid : Methanol = 20:1)

### Beispiel VIII

### 3-Brom-2-cyanpyridin

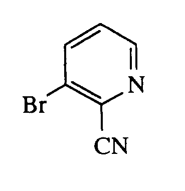

Eine Lösung von Beispiel VII (22 g; 0,12 mol), Trimethylsilylcyanid (45 ml; 0,36 mmol) und Triethylamin (33 ml; 0,24 mol) in Acetonitril (120 ml) wird 4 h zum Rückfluß erhitzt, eingeengt und auf 3N-Natriumcarbonat-Lösung gegossen. Extraktion mit Methylenchlorid sowie Trocknen und Einengen der organischen Phasen liefern nach Umkristallisation aus Hexan / Ethylacetat 17 g eines Feststoffs (79% der Theorie).
Fp.: 92°C
R_{f} = 0,31 (Hexan : Essigester = 3:1)

### Beispiel IX

### 2-Cyan-3-hex-1-inylpyridin

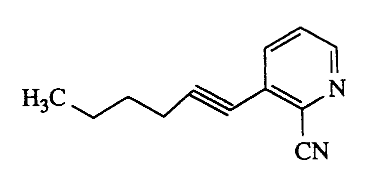

Im Autoklaven werden die Verbindung aus Beispiel VIII (4,8 g; 26 mmol), 1-Hexin (3,6 ml; 31 mmol), Bis-(triphenylphosphin)-Palladium(II)-chlorid (0,42 g; 0,60 mmol) und Kupfer(I)-jodid (0,21 g; 1,1 mmol) mit Stickstoff gespült und 5 h auf 120°C erhitzt. Verteilung des Reaktionsgemisches zwischen Wasser und Ether, sowie Trocknen und Einengen der organischen Phase liefert nach Kieselgelchromatographie (Hexan : Essigester = 4:1) 0,76 g eines Öls (16% der Theorie).
R_{f} = 0,60 (Hexan : Essigester = 3:1)

### Beispiel X

### 4-(Brommethyl)benzol-sulfochlorid

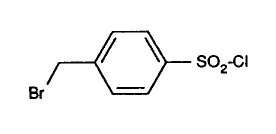

38,1 g (0,2 mol) 4-Methylbenzolsulfonylchlorid werden in 300 ml Tetrachlorkohlenstoff gelöst und mit 35,6 g (0,2 mol) N-Bromsuccinimid versetzt und nach Zugabe von 0,2 g (1,2 mmol) Azobisisoburyronitril (ABU) für 4 h unter Rückfluß erhitzt. Nach dem Abkühlen werden die Feststoffe abfiltriert und das Filtrat vom Lösemittel befreit. Flash-Chromatographie (Petrolether / Toluol 4:1, 50 »m Korngröße) und anschließende Umkristallisation aus 100 ml Cyclohexan ergehen 24,0 g (45% der Theorie) der Titelverbindung.
R_{f} = 0,75 (Toluol)

### Beispiel XI

### 4-(Brommethyl)-3-chlorbenzolsulfochlorid

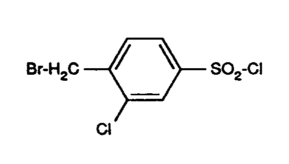

45,9 g (0,2 mol) 3-Chlor-4-methylbenzolsulfonsäure Natriumsalz werden mit 83,3 g (0,4 mol) Phosphorpentachlorid gemischt und 30 min bei 140°C Ölbadtemperatur erhitzt. In der Hitze wird mit 500 ml Toluol versetzt, die entstandene Lösung bis zum Sieden erhitzt und nach dem Abkühlen auf Eis gegeben. Die organische Phase wird abgetrennt und mit Wasser gewaschen (2 x 200 ml). Nach dem Trocknen über MgSO₄ wird filtriert und alles Flüchtige im Vakuum abgezogen. Der erhaltene Rückstand wird durch Flashchromatographie (Petrolether / Toluol 4:1, 50 » Korngröße) gereinigt. Man erhält 24,9 g eines Produkts, das sofort weiter umgesetzt wird:
Man nimmt in 200 ml Tetrachlorkohlenstoff auf und erhitzt nach Zugabe von 19,6 g (0,11 mol) N-Bromsuccinimid und 0,1 g (0,6 mmol) ABN für 6 h unter Rückfluß. Nach dem Abkühlen werden die Feststoffe abfiltriert und das Filtrat vom Lösemittel befreit. Flashchromatographie (Petrolether / Toluol 4:1, 50 » Korngröße) ergibt 21,2 g (35%) der Titelverbindung.
R_{f} = 0,32 (Petrolether / Dichlormethan 4:1)

### Beispiel XII

### 4-(Brommethyl)-benzolsulfonyl-N-pyrrolidinid

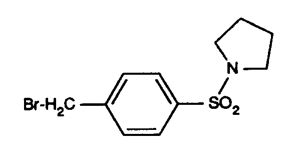

5,3 g (0,02 mol) der Verbindung aus Beispiel X werden in 200 ml Dichlormethan und 4,0 g (0,04 mol) Triethylamin gelöst und nach Zugabe von 1,4 g (0,02 mol) Pyrrolidin in 50 ml Dichlormethan bei 0°C für 1 h bei 0°C nachgerührt. Man extrahiert mit 2 N HCl (2 x 100 ml), H₂O (2 x 100 ml), trocknet über MgSO₄, filtriert und verdampft alle flüchtigen Anteile im Vakuum.
Ausbeute: 5,4g (89% der Theorie)
R_{f} = 0,09 (Toluol)

### Beispiel XIII

### 4-(Brommethyl)benzolsulfonyl-N-piperidinid

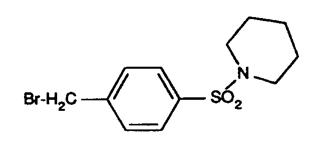

In Analogie zur Vorschrift des Beispiels XII erhält man aus 1,1 g (4 mmol) der Verbindung aus Beispiel I und 0,34 g (4 mmol) Piperidin 1,0 g (81% der Theorie) der Titelverbindung.
R_{f} = 0,14 (Toluol)

### Beispiel XIV

### (S)-4-(Brommethyl)-benzolsulfonyl-N-2-(tert.butoxycarbonyl)pyrrolidinid

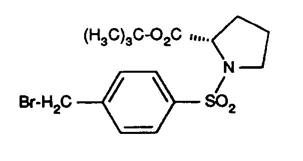

In Analogie zur Vorschrift des Beispiels XII erhält man aus 7,25 g (27 mmol) der Verbindung aus Beispiel I und 4,6 g (27 mmol) S-Prolin-tert.butylester 9,1 g (84% der Theorie) der Titelverbindung.
R_{f} = 0,66 (Petrolether / Essigester 7:3)

### Beispiel XV

### rac-4-(Brommethyl)-benzolsulfonyl-N-2-(tert.butoxycarbonyl)piperidninid

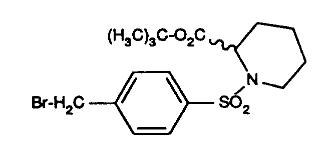

In Analogie zur Vorschrift des Beispiels XII erhält man aus 8,0 g (30 mmol) der Verbindung aus Beispiel I und 5,5 g (30 mmol) rac-Piperolinsäure-tert.butylester 7,4 g (59% der Theorie) der Titelverbindung.
R_{f}= 0,53 (Petrolether / Essigester 5:1)

### Beispiel XIV

### (S)-4-(Brommethyl)-3-chlorbenzolsulfonyl-N-2-(tert.butoxy carbonyl)pyrrolidinid

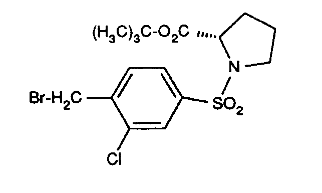

In Analogie zur Vorschrift des Beispiels XII erhält man aus 10,0 g (33 mmol) der Verbindung aus Beispiel II und 5,7 g (33 mmol) S-Prolin-tert.butylester 13,9 g (96% der Theorie) der Titelverbindung.
R_{f} = 0,55 (Petrolether / Essigester 7:3)

### Beispiel XVII

### rac-4-(Brommethyl)-3-chlorbenzolsulfonyl-N-2-(tert.butoxycarbonyl)piperidinid

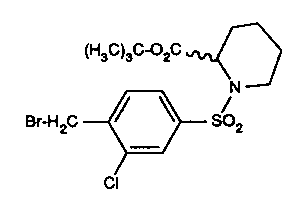

In Analogie zur Vorschrift des Beispiels XII erhält man aus 10,0 g (33 mmol) der Verbindung aus Beispiel II und 6,1 g (33 mmol) rac-Pipecolinsäure-tert.butylester 14,6 g (98% der Theorie) der Titelverbindung.
R_{f} = 0,6 (Petrolether / Essigester 7:3)

### Herstellungsbeispiele

### Beispiel 1

### N-4-[3-Butyl-1,2-dihydro-1-oxo-isochinolin-2-yl-methyl]-3-chlorphenylsulfonylprolin tert.-Butylester

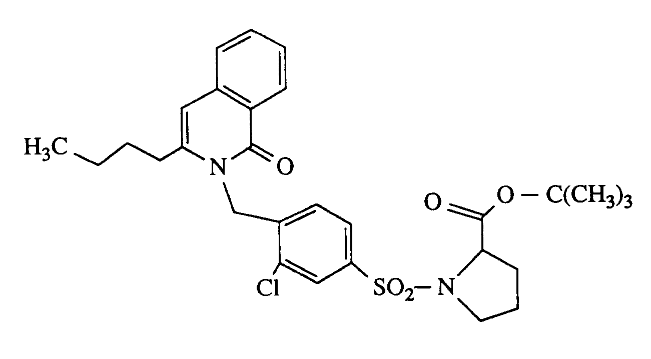

Die Verbindung aus Beispiel II (710 mg; 1,35 mmol) wird in Dimethoxyethan (25 ml) gelöst und mit dem Produkt aus Beispiel XVI (1,86 g; 4,24 mmol) und Caesiumcarbonat (1,38 g; 4,24 mmol) versetzt und über Nacht gerührt. Nach zweimaliger Zugabe der gleichen Menge Caesiumcarbonat und weiteren zwei Tagen Rühren wird die Reaktionslösung eingeengt und zwischen Wasser und Essigester verteilt Die organische Phase wird mit Natriumsulfat getrocknet, eingeengt, und der Rückstand wird über Kieselgel chromatographiert (Hexan:Essigester = 4:1), um 0,80 g eines Feststoffs zu ergehen (40 % der Theorie).
R_{f} = 0,28 (Hexan:Essigester = 3:1).

### Beispiel 2

### N-4-[3-Butyl-1,2-dihydro-1-oxo-isochinolin-2-yl-methyl]-3-chlorphenylsulfonylprolin

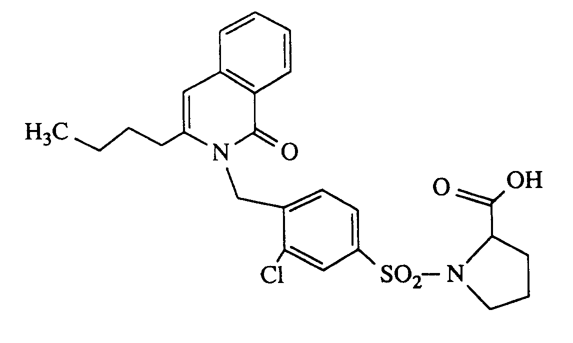

Das Produkt aus Beispiel I (756 mg; 1,35 mmol) wird in Methylenchlorid (40 ml) gelöst und mit Trifluoressigsäure (40 ml) versetzt. Nach 2 Stunden wird die Reaktionslösung eingeengt, der Rückstand in Essigester gelöst, mit 1 N Kaliumhydrogensulfat-Lösung gewaschen und mit Natriumsulfat getrocknet. Einengen liefert 0,68 g eines Feststoffs (100 % der Theorie).
R_{f} = 0,34 (Methylenchlorid:Methanol = 10:1).

## Patentansprüche (Patentansprüche für folgende(n) Vertragsstaat(en): AT, BE, CH, DE, DK, FR, GB, GR, IE, IT, LI, LU, MC, NL, PT, SE)

1. Sulfonylbenzyl-substituierte Benzo- und Pyridopyridone der allgemeinen Formel 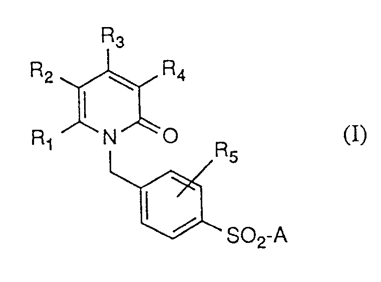 in welcher
R¹und R² gleich oder verschieden sind und für Wasserstoff, Cyano oder
für geradkettiges oder verzweigtes Alkyl, Alkenyl oder Alkinyl mit jeweils bis zu 8 Kohlenstoffatomen stehen, die gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen oder Phenyl substituiert sind, oder
für Cycloalkyl mit 3 bis 6 Kohlenstoffatomen stehen,
für geradkettiges oder verzweigtes Acyl oder Alkoxycarbonyl mit jeweils bis zu 8 Kohlenstoffatomen, Benzyloxycarbonyl oder Carboxy stehen, oder
für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder
für die Gruppe der Formel -CO-NR⁶R⁷, B-R⁸ oder NR⁹R¹⁰ stehen,
worin
R⁶ und R⁷ gleich oder verschieden sind und Wasserstoff, Phenyl, geradkettiges oder verzweigtes Alkyl mit bis zu 6 Kohlenstoffatomen oder Benzyl bedeuten,
B ein Sauerstoff- oder Schwefelatom bedeutet,
R⁸ geradkettiges oder verzweigtes Alkyl mit bis zu 8 Kohlenstoffatomen bedeutet,
R⁹ und R¹⁰ gleich oder verschieden sind und die oben angegebene Bedeutung von R⁶ und R⁷ haben, oder
R⁹ oder R¹⁰ die -SO₂R¹¹-Gruppe bedeutet,
worin
R¹¹ geradkettiges oder verzweigtes Alkyl mit bis zu 6 Kohlenstoffatomen, Benzyl oder Phenyl bedeutet, die gegebenenfalls durch Methyl substituiert sind,
R³ und R⁴ unter Einbezug der Doppelbindung einen Phenyl- oder Pyridylring ausbilden, der gegebenenfalls bis zu 3-fach gleich oder verschieden durch Hydroxy, Formyl, Carboxy, Halogen, geradkettiges oder verzweigtes Acyl oder Alkoxycarbonyl mit jeweils bis zu 8 Kohlenstoffatomen, geradkettiges oder verzweigtes Perfluoralkyl mit bis zu 6 Kohlenstoffatomen oder durch geradkettiges oder verzweigtes Alkyl mit bis zu 8 Kohlenstoffatomen substituiert ist, das seinerseits durch Hydroxy oder geradkettiges oder verzweigtes Alkoxy mit bis zu 6 Kohlenstoffatomen substituiert sein kann, oder durch die Gruppe der Formel -CONR⁶R⁷ substituiert ist,
worin
R⁶ und R⁷ die oben angegebene Bedeutung haben,
R⁵ für Wasserstoff, Nitro, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder
für eine Gruppe der Formel -OR¹², -NR¹³R¹⁴ oder -NR¹³-CO-R¹⁵ steht,
worin
R¹² Wasserstoff, geradkettiges oder verzweigtes Alkyl mit bis zu 8 Kohlenstoffatomen oder Phenyl bedeutet,
R¹³ und R¹⁴ gleich oder verschieden sind und die oben angegebene Bedeutung von R⁶ und R⁷ haben
und
R¹⁵ geradkettiges oder verzweigtes Alkyl mit bis zu 6 Kohlenstoffatomen oder Phenyl bedeutet
A für einen über das Stickstoffatom gebundenen 3- bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel
-NR^{9'}R^{10'}, -SO₃H , 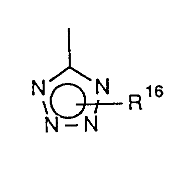 oder -CO-R¹⁷
substituiert ist,
worin
R^{9'} und R^{10'} die oben angegebene Bedeutung von R⁹ und R¹⁰ haben und mit dieser gleich oder verschieden sind,
R¹⁶ Wasserstoff, geradkettiges oder verzweigtes Alkyl mit bis zu 6 Kohlenstoffatomen oder Triphenylmethyl bedeutet
und
R¹⁷ Hydroxy, geradkettiges oder verzweigtes Alkoxy mit bis zu 8 Kohlenstoffatomen, Phenoxy oder eine Gruppe der Formel -NR¹⁸R¹⁹ bedeutet,
worin
R¹⁸ und R¹⁹ gleich oder verschieden sind und Wasserstoff oder geradkettiges oder verzweigtes Alkyl mit bis zu 6 Kohlenstoffatomen oder Phenyl bedeuten,
und deren Salze.

2. Sulfonylbenzyl-substituierte Benzo- und Pyridopyridone nach Anspruch 1, wobei
R¹und R² gleich oder verschieden sind und für Wasserstoff, Cyano, oder
für geradkettiges oder verzweigtes Alkyl, Alkenyl oder Alkinyl mit jeweils bis zu 6 Kohlenstoffatomen stehen, die gegebenenfalls durch Cyclopropyl, Cyclopentyl, Cyclohexyl, Hydroxy oder durch geradkettiges oder verzweigtes Alkoxy mit bis zu 4 Kohlenstoffatomen oder Phenyl substituiert sind, oder
für Cyclopropyl, Cyclopentyl oder Cyclohexyl stehen,
für geradkettiges oder verzweigtes Acyl oder Alkoxycarbonyl mit jeweils bis zu 6 Kohlenstoffatomen, Benzyloxycarbonyl oder Carboxy stehen, oder
für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder
für eine Gruppe der Formel -CO-NR⁶R⁷, B-R⁸ oder -NR⁹R¹⁰ stehen,
worin
R⁶ und R⁷ gleich oder verschieden sind und Wasserstoff, Phenyl, geradkettiges oder verzweigtes Alkyl mit bis zu 4 Kohlenstoffatomen oder Benzyl bedeuten,
B ein Sauerstoff- oder Schwefelatom bedeutet,
R⁸ geradkettiges oder verzweigtes Alkyl mit bis zu 6 Kohlenstoffatomen bedeutet,
R⁹ und R¹⁰ gleich oder verschieden sind und die oben angegebene Bedeutung von R⁶ und R⁷ haben oder R⁹ oder R¹⁰ die -SO₂R¹¹-Gruppe bedeutet,
worin
R¹¹ geradkettiges oder verzweigtes Alkyl mit bis zu 4 Kohlenstoffatomen,Phenyl oder Tolyl bedeutet,
R³ und R⁴ gemeinsam unter Einbezug der Doppelbindung einen Phenyl- oder Pyridylring ausbilden, der gegebenenfalls bis zu 2-fach gleich oder verschieden durch Hydroxy, Formyl, Carboxy, Fluor, Chlor, Brom, geradkettiges oder verzweigtes Acyl oder Alkoxycarbonyl mit jeweils bis zu 6 Kohlenstoffatomen, geradkettiges oder verzweigtes Perfluoralkyl mit bis zu 4 Kohlenstoffatomen oder durch geradkettiges oder verzweigtes Alkyl mit bis zu 6 Kohlenstoffatomen substituiert ist, das seinerseits durch Hydroxy oder durch geradkettiges oder verzweigtes Alkoxy mit bis zu 4 Kohlenstoffatomen substituiert sein kann, oder durch die Gruppe der Formel -CONR⁶R⁷ substituiert ist,
worin
R⁶ und R⁷ die oben angegebene Bedeutung haben,
R⁵ für Wasserstoff, Nitro, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht, oder
für eine Gruppe der Formel -OR¹², -NR¹³R¹⁴ oder -NR¹³-CO-R¹⁵ steht,
worin
R¹² Wasserstoff oder geradkettiges oder verzweigtes Alkyl mit bis zu 6 Kohlenstoffatomen bedeutet,
R¹³ und R¹⁴ gleich oder verschieden sind und die oben angegebene Bedeutung von R⁶ und R⁷ haben
und
R¹⁵ geradkettiges oder verzweigtes Alkyl mit bis zu 6 Kohlenstoffatomen oder Phenyl bedeutet
A für über das Stickstoffatom gebundenes Piperidyl, Pyrrolidinyl oder Morpholinyl steht, die gegebenenfalls durch einen Rest der Formel
-NR^{9'}R^{10'}, -SO₃H , 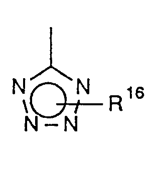 oder -CO-R¹⁷
substituiert sind,
worin
R^{9'} und R^{10'} die oben angegebene Bedeutung von R⁹ und R¹⁰ haben und mit dieser gleich oder verschieden sind,
R¹⁶ Wasserstoff, geradkettiges oder verzweigtes Alkyl mit bis zu 4 Kohlenstoffatomen oder Triphenylmethyl bedeutet
und
R¹⁷ Hydroxy, geradkettiges oder verzweigtes Alkoxy mit bis zu 6 Kohlenstoffatomen, Phenoxy oder eine Gruppe der Formel -NR¹⁸R¹⁹ bedeutet,
worin
R¹⁸ und R¹⁹ gleich oder verschieden sind und Wasserstoff oder geradkettiges oder verzweigtes Alkyl mit bis zu 4 Kohlenstoffatomen bedeuten,
und deren Salze.

3. Sulfonylbenzyl-substituierte Benzo- und Pyridopyridone nach Anspruch 1, worin
R¹ und R² gleich oder verschieden sind und für Wasserstoff, Cyano oder
für geradkettiges oder verzweigtes Alkyl mit bis zu 4 Kohlenstoffatomen stehen, das gegebenenfalls durch Cyclopropyl substituiert ist oder
für Cyclopropyl oder Phenyl stehen, oder
für geradkettiges oder verzweigtes Acyl oder Alkoxycarbonyl mit jeweils bis zu 4 Kohlenstoffatomen, Benzyloxycarbonyl oder Carboxy stehen, oder
für eine Gruppe der Formel -CO-NR⁶R⁷, B-R⁸ oder NR⁹R¹⁰ stehen,
worin
R⁶ und R⁷ gleich oder verschieden sind und Wasserstoff, Phenyl, Ethyl oder Benzyl bedeuten,
B ein Sauerstoff- oder Schwefelatom bedeutet,
R⁸ geradkettiges oder verzweigtes Alkyl mit bis zu 4 Kohlenstoffatomen bedeutet,
R⁹ und R¹⁰ gleich oder verschieden sind und die oben angegebene Bedeutung von R⁶ und R⁷ haben, oder
R⁹ oder R¹⁰ die -SO₂R¹¹-Gruppe bedeutet,
worin
R¹¹ Methyl, Phenyl oder Tolyl bedeutet,
R³ und R⁴ gemeinsam unter Einbezug der Doppelbindung einen ankondensierten Phenyl- oder Pyridylring ausbilden, der gegebenenfalls bis zu 2-fach gleich oder verschieden durch Hydroxy, Carboxy, Fluor, Chlor, geradkettiges oder verzweigtes Acyl oder Alkoxycarbonyl mit jeweils bis zu 4 Kohlenstoffatomen oder geradkettiges oder verzweigtes Perfluoralkyl mit bis zu 3 Kohlenstoffatomen oder durch geradkettiges oder verzweigtes Alkyl mit bis zu 4 Kohlenstoffatomen substituiert ist, das seinerseits durch Hydroxy oder durch geradkettiges oder verzweigtes Alkoxy mit bis zu 3 Kohlenstoffatomen substituiert sein kann
oder
durch die Gruppe der Formel -CO-NR⁶R⁷ substituiert ist,
worin
R⁶ und R⁷ die oben angegebene Bedeutung haben,
R⁵ für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht, oder
für eine Gruppe der Formel -OR¹², -NR¹³R¹⁴ oder -NR¹³-CO-R¹⁵ steht,
worin
R¹² Wasserstoff oder geradkettiges oder verzweigtes Alkyl mit bis zu 4 Kohlenstoffatomen bedeutet,
R¹³ und R¹⁴ gleich oder verschieden sind und die oben angegebene Bedeutung von R⁶ und R⁷ haben
und
R¹⁵ geradkettiges oder verzweigtes Alkyl mit bis zu 6 Kohlenstoffatomen oder Phenyl bedeutet
A für über das Stickstoffatom gebundenes Piperidyl oder Pyrrolidinyl steht, die gegebenenfalls durch einen Rest der Formel
-NR^{9'}R^{10'}, -SO₃H , 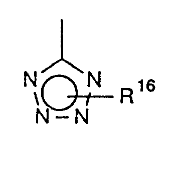 oder -CO-R¹⁷
substituiert sind,
worin
R^{9'} und R^{10'} die oben angegebene Bedeutung von R⁹ und R¹⁰ haben und mit dieser gleich oder verschieden sind,
R¹⁶ Wasserstoff, Methyl, Ethyl oder Triphenylmethyl bedeutet
und
R¹⁷ Hydroxy, geradkettiges oder verzweigtes Alkoxy mit bis zu 4 Kohlenstoffatomen, Phenoxy oder eine Gruppe der Formel -NR¹⁸R¹⁹ bedeutet,
worin
R¹⁸ und R¹⁹ gleich oder verschieden sind und Wasserstoff oder geradkettiges oder verzweigtes Alkyl mit bis zu 3 Kohlenstoffatomen bedeuten
und deren Salze.

4. Sulfonylbenzyl-substituierte Benzo- und Pyridopyridone nach Anspruch 1 zur therapeutischen Anwendung.

5. Verfahren zur Herstellung von Sulfonylbenzyl-substituierten Benzo- und Pyridopyridonen nach Anspruch 1, dadurch gekennzeichnet, daß man
Pyridone der allgemeinen Formel (II) 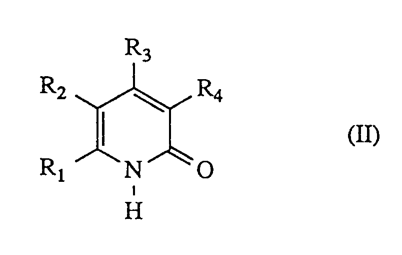 in welcher
R¹, R², R³ und R⁴ die oben angegebene Bedeutung haben,
mit Verbindungen der allgemeinen Formel (III) 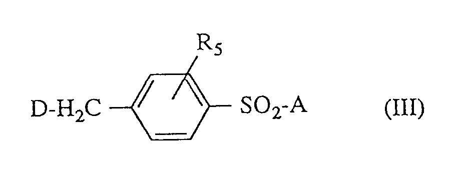 in welcher
R⁵ und A die oben angegebene Bedeutung haben
und
D für Halogen, vorzugsweise für Brom steht,
in organischen Lösemitteln und in Anwesenheit einer Base und gegebenenfalls eines Katalysators umsetzt,
und im Fall, daß R¹⁶ nicht für Wasserstoff steht, eine Alkylierung anschließt,
und im Fall der Säuren (R¹⁷ = OH) die entsprechenden Ester verseift,
und im Fall der Ester oder Amide, gegebenenfalls über eine aktivierte Carbonsäurestufe, eine Veresterung bzw. Amidierung anschließt,
und sowohl die Substituenten R¹, R² und R⁵ als auch die Substituenten des Phenyl- und Pyridylrings (R³/R⁴) nach üblichen Methoden variiert.

6. Arzneimittel enthaltend Sulfonylbenzyl-substituierte Benzo- und Pyridopyridone nach Anspruch 1.

7. Arzneimittel nach Anspruch 6 zur Behandlung von arterieller Hypertonie und Atherosklerose.

8. Verwendung von Sulfonylbenzyl-substituierten Benzo- und Pyridopyridonen nach Anspruch 1 zur Herstellung von Arzneimitteln.

## Patentansprüche (Patentansprüche für folgende(n) Vertragsstaat(en): ES)

1. Verfahren zur Herstellung von Sulfonylbenzyl-substituierten Benzol und Pyridopyridonen der allgemeinen Formel 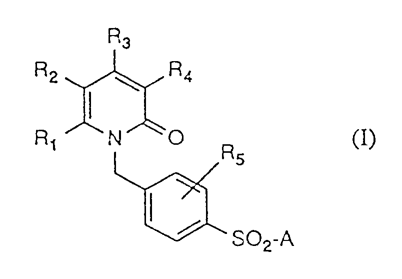 in welcher
R¹und R² gleich oder verschieden sind und für Wasserstoff, Cyano oder
für geradkettiges oder verzweigtes Alkyl, Alkenyl oder Alkinyl mit jeweils bis zu 8 Kohlenstoffatomen stehen, die gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen oder Phenyl substituiert sind, oder
für Cycloalkyl mit 3 bis 6 Kohlenstoffatomen stehen,
für geradkettiges oder verzweigtes Acyl oder Alkoxycarbonyl mit jeweils bis zu 8 Kohlenstoffatomen, Benzyloxycarbonyl oder Carboxy stehen, oder
für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder
für die Gruppe der Formel -CO-NR⁶R⁷, B-R⁸ oder -NR⁹R¹⁰ stehen,
worin
R⁶ und R⁷ gleich oder verschieden sind und Wasserstoff, Phenyl, geradkettiges oder verzweigtes Alkyl mit bis zu 6 Kohlenstoffatomen oder Benzyl bedeuten,
B ein Sauerstoff- oder Schwefelatom bedeutet,
R⁸ geradkettiges oder verzweigtes Alkyl mit bis zu 8 Kohlenstoffatomen bedeutet,
R⁹ und R¹⁰ gleich oder verschieden sind und die oben angegebene Bedeutung von R⁶ und R⁷ haben, oder
R⁹ oder R¹⁰ die -SO₂R¹¹-Gruppe bedeutet,
worin
R¹¹ geradkettiges oder verzweigtes Alkyl mit bis zu 6 Kohlenstoffatomen, Benzyl oder Phenyl bedeutet, die gegebenenfalls durch Methyl substituiert sind,
R³ und R⁴ unter Einbezug der Doppelbindung einen Phenyl- oder Pyridylring ausbilden, der gegebenenfalls bis zu 3-fach gleich oder verschieden durch Hydroxy, Formyl, Carboxy, Halogen, geradkettiges oder verzweigtes Acyl oder Alkoxycarbonyl mit jeweils bis zu 8 Kohlenstoffatomen, geradkettiges oder verzweigtes Perfluoralkyl mit bis zu 6 Kohlenstoffatomen oder durch geradkettiges oder verzweigtes Alkyl mit bis zu 8 Kohlenstoffatomen substituiert ist, das seinerseits durch Hydroxy oder geradkettiges oder verzweigtes Alkoxy mit bis zu 6 Kohlenstoffatomen substituiert sein kann, oder durch die Gruppe der Formel -CONR⁶R⁷ substituiert ist,
worin
R⁶ und R⁷ die oben angegebene Bedeutung haben,
R⁵ für Wasserstoff, Nitro, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder
für eine Gruppe der Formel -OR¹², -NR¹³R¹⁴ oder -NR¹³-CO-R¹⁵ steht,
worin
R¹² Wasserstoff, geradkettiges oder verzweigtes Alkyl mit bis zu 8 Kohlenstoffatomen oder Phenyl bedeutet,
R¹³ und R¹⁴ gleich oder verschieden sind und die oben angegebene Bedeutung von R⁶ und R⁷ haben
und
R¹⁵ geradkettiges oder verzweigtes Alkyl mit bis zu 6 Kohlenstoffatomen oder Phenyl bedeutet
A für einen über das Stickstoffatom gebundenen 3- bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel
-NR^{9'}R^{10'}, -SO₃H , 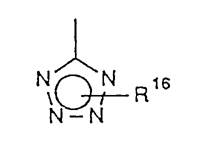 oder -CO-R¹⁷
substituiert ist,
worin
R^{9'} und R^{10'} die oben angegebene Bedeutung von R⁹ und R¹⁰ haben und mit dieser gleich oder verschieden sind,
R¹⁶ Wasserstoff, geradkettiges oder verzweigtes Alkyl mit bis zu 6 Kohlenstoffatomen oder Triphenylmethyl bedeutet
und
R¹⁷ Hydroxy, geradkettiges oder verzweigtes Alkoxy mit bis zu 8 Kohlenstoffatomen, Phenoxy oder eine Gruppe der Formel -NR¹⁸R¹⁹ bedeutet,
worin
R¹⁸ und R¹⁹ gleich oder verschieden sind und Wasserstoff oder geradkettiges oder verzweigtes Alkyl mit bis zu 6 Kohlenstoffatomen oder Phenyl bedeuten,
und deren Salze
**dadurch gekennzeichnet,** daß man
Pyridone der allgemienen Formel (II) 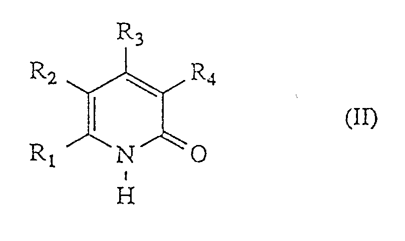 in welcher
R¹, R², R³ und R⁴ die oben angegebene Bedeutung haben,
mit Verbindungen der allgemeinen Formel (III) 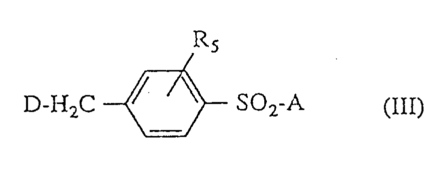 in welcher
R⁵ und A die oben angegebene Bedeutung haben
und
D für Halogen, vorzugsweise für Brom steht,
in organischen Lösemitteln und in Anwesenheit einer Base und gegebenenfalls eines Katalysators umsetzt,
und im Fall, daß R¹⁶ nicht für Wasserstoff steht, eine Alkylierung anschließt,
und im Fall der Säuren (R¹⁷ = OH) die entsprechenden Ester verseift,
und im Fall der Ester oder Amide, gegebenenfalls über eine aktivierte Carbonsäurestufe, eine Veresterung bzw. Amidierung anschließt,
und sowohl die Substituenten R¹, R² und R⁵ als auch die Substituenten des Phenyl- und Pyridylrings (R³/R⁴) nach üblichen Methoden variiert

## Claims (Claims for the following Contracting State(s): AT, BE, CH, DE, DK, FR, GB, GR, IE, IT, LI, LU, MC, NL, PT, SE)

1. Sulphonylbenzyl-substituted benzo- and pyridopyridones of the general formula 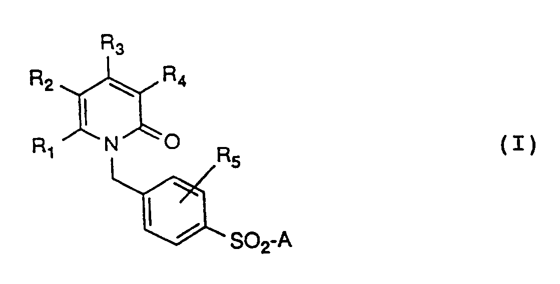 in which
R¹ and R² are identical or different and represent hydrogen or cyano, or
represent straight-chain or branched alkyl, alkenyl or alkinyl having in each case up to 8 carbon atoms, which are optionally substituted by cycloalkyl having 3 to 6 carbon atoms, hydroxyl or by straight-chain or branched alkoxy having up to 6 carbon atoms or phenyl, or
represent cycloalkyl having 3 to 6 carbon atoms, or
represent straight-chain or branched acyl or alkoxycarbonyl having in each case up to 8 carbon atoms, benzyloxycarbonyl or carboxyl, or
represent phenyl, which is optionally substituted up to 3 times in an identical or different manner by halogen, nitro, cyano, hydroxyl, hydroxymethyl, trifluoromethyl or trifluoromethoxy or by straight-chain or branched alkyl or alkoxy having in each case up to 6 carbon atoms, or
represent the group of the formula -CO-NR⁶R⁷, B-R⁸ or -NR⁹R¹⁰,
wherein
R⁶ and R⁷ are identical or different and denote hydrogen, phenyl, straight-chain or branched alkyl having up to 6 carbon atoms or benzyl,
B denotes an oxygen or sulphur atom,
R⁸ denotes straight-chain or branched alkyl having up to 8 carbon atoms,
R⁹ and R¹⁰ are identical or different and have the abovementioned meaning of R⁶ and R⁷ or
R⁹ or R¹⁰ denotes the -SO₂R¹¹ group,
wherein
R¹¹ denotes straight-chain or branched alkyl having up to 6 carbon atoms, benzyl or phenyl, which are optionally substituted by methyl,
R³ and R⁴, including the double bond, form a phenyl or pyridyl ring which is optionally substituted up to 3 times in an identical or different manner by hydroxyl, formyl, carboxyl, halogen, straight-chain or branched acyl or alkoxycarbonyl having in each case up to 8 carbon atoms or straight-chain or branched perfluoroalkyl having up to 6 carbon atoms or by straight-chain or branched alkyl having up to 8 carbon atoms, which in its turn can be substituted by hydroxyl or straight-chain or branched alkoxy having up to 6 carbon atoms, or is substituted by the group of the formula -CONR⁶R⁷,
wherein
R⁶ and R⁷ have the abovementioned meaning,
R⁵ represents hydrogen, nitro, halogen or straight-chain or branched alkyl having up to 8 carbon atoms, or
represents straight-chain or branched perfluoroalkyl having up to 6 carbon atoms, or
represents a group of the formula -OR¹², -NR¹³R¹⁴ or -NR¹³-CO-R¹⁵,
wherein
R¹² denotes hydrogen, straight-chain or branched alkyl having up to 8 carbon atoms or phenyl,
R¹³ and R¹⁴ are identical or different and have the abovementioned meaning of R⁶ and R⁷ and
R¹⁵ denotes straight-chain or branched alkyl having up to 6 carbon atoms or phenyl,
A represents a 3- to 8-membered saturated heterocyclic ring which is bonded via the nitrogen atom and has up to 2 further hetero atoms from the series comprising S, N and O, and is optionally substituted up to twice in an identical or different manner by a radical of the formula
-NR^{9'}R^{10'}, -SO₃H, 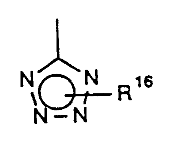 or -CO-R¹⁷,
wherein
R^{9'}and R^{10'} have the abovementioned meaning of R⁹ and R¹⁰ and are identical to or different from these,
R¹⁶ denotes hydrogen, straight-chain or branched alkyl having up to 6 carbon atoms or triphenylmethyl
and
R¹⁷ denotes hydroxyl, straight-chain or branched alkoxy having up to 8 carbon atoms, phenoxy or a group of the formula -NR¹⁸R¹⁹,
wherein
R¹⁸ and R¹⁹ are identical or different and denote hydrogen, straight-chain or branched alkyl having up to 6 carbon atoms or phenyl,
and salts thereof.

2. Sulphonylbenzyl-substituted benzo- and pyridopyridones according to Claim 1, wherein
R¹ and R² are identical or different and represent hydrogen or cyano, or
represent straight-chain or branched alkyl, alkenyl or alkinyl having in each case up to 6 carbon atoms, which are optionally substituted by cyclopropyl, cyclopentyl, cyclohexyl or hydroxyl or by straight-chain or branched alkoxy having up to 4 carbon atoms or phenyl, or
represent cyclopropyl, cyclopentyl or cyclohexyl, or
represent straight-chain or branched acyl or alkoxycarbonyl having in each case up to 6 carbon atoms, benzyloxycarbonyl or carboxyl, or
represent phenyl, which is optionally substituted up to twice in an identical or different manner by fluorine, chlorine, bromine, trifluoromethyl, trifluoromethoxy or hydroxymethyl or by straight-chain or branched alkyl or alkoxy having in each case up to 4 carbon atoms, or
represent a group of the formula -CO-NR⁶R⁷, B-R⁸ or -NR⁹R¹⁰,
wherein
R⁶ and R⁷ are identical or different and denote hydrogen, phenyl, straight-chain or branched alkyl having up to 4 carbon atoms or benzyl,
B denotes an oxygen or sulphur atom,
R⁸ denotes straight-chain or branched alkyl having up to 6 carbon atoms,
R⁹ and R¹⁰ are identical or different and have the abovementioned meaning of R⁶ and R⁷, or R⁹ or R¹⁰ denotes the -SO₂R¹¹ group,
wherein
R¹¹ denotes straight-chain or branched alkyl having up to 4 carbon atoms, phenyl or tolyl,
R³ and R⁴, together and including the double bond, form a phenyl or pyridyl ring which is optionally substituted up to twice in an identical or different manner by hydroxyl, formyl, carboxyl, fluorine, chlorine, bromine, straight-chain or branched acyl or alkoxycarbonyl having in each case up to 6 carbon atoms or straight-chain or branched perfluoroalkyl having up to 4 carbon atoms or by straight-chain or branched alkyl having up to 6 carbon atoms, which in its turn can be substituted by hydroxyl or by straight-chain or branched alkoxy having up to 4 carbon atoms, or is substituted by the group of the formula -CONR⁶R⁷,
wherein
R⁶ and R⁷ have the abovementioned meaning,
R⁵ represents hydrogen, nitro, fluorine, chlorine, bromine or straight-chain or branched alkyl having up to 6 carbon atoms, or
represents straight-chain or branched perfluoroalkyl having up to 4 carbon atoms, or
represents a group of the formula -OR¹², -NR¹³R¹⁴ or -NR¹³-CO-R¹⁵,
wherein
R¹² denotes hydrogen or straight-chain or branched alkyl having up to 6 carbon atoms, the abovementioned meaning of R⁶ and R⁷
and
R¹⁵ denotes straight-chain or branched alkyl having up to 6 carbon atoms, or phenyl,
A represents piperidyl, pyrrolidinyl or morpholinyl which are bonded via the nitrogen atom and are optionally substituted by a radical of the formula
-NR^{9'}R^{10'}, -SO₃H, 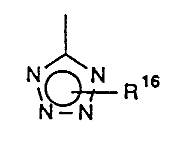 or -CO-R¹⁷,
wherein
R^{9'} and R^{10'} have the abovementioned meaning of R⁹ and R¹⁰ and are identical to or different from these,
R¹⁶ denotes hydrogen, straight-chain or branched alkyl having up to 4 carbon atoms or triphenylmethyl
and
R¹⁷ denotes hydroxyl, straight-chain or branched alkoxy having up to 6 carbon atoms, phenoxy or a group of the formula -NR¹⁸R¹⁹,
wherein
R¹⁸ and R¹⁹ are identical or different and denote hydrogen or straight-chain or branched alkyl having up to 4 carbon atoms,
and salts thereof.

3. Sulphonylbenzyl-substituted benzo- and pyridopyridones according to Claim 1,
wherein
R¹ and R² are identical or different and represent hydrogen or cyano, or
represent straight-chain or branched alkyl having up to 4 carbon atoms, which is optionally substituted by cyclopropyl, or
represent cyclopropyl or phenyl, or
represent straight-chain or branched acyl or alkoxycarbonyl having in each case up to 4 carbon atoms, benzyloxycarbonyl or carboxyl, or
represent a group of the formula -CO-NR⁶R⁷, B-R⁸ or -NR⁹R¹⁰,
wherein
R⁶ and R⁷ are identical or different and denote hydrogen, phenyl, ethyl or benzyl,
B denotes an oxygen or sulphur atom,
R⁸ denotes straight-chain or branched alkyl having up to 4 carbon atoms,
R⁹ and R¹⁰ are identical or different and have the abovementioned meaning of R⁶ and R⁷, or
R⁹ or R¹⁰ denotes the -SO₂R¹¹ group,
wherein
R¹¹ denotes methyl, phenyl or tolyl,
R³ and R⁴, together and including the double bond, form a fused-on phenyl or pyridyl ring which is optionally substituted up to twice in an identical or different manner by hydroxyl, carboxyl, fluorine, chlorine, straight-chain or branched acyl or alkoxycarbonyl having in each case up to 4 carbon atoms or straight-chain or branched perfluoroalkyl having up to 3 carbon atoms or by straight-chain or branched alkyl having up to 4 carbon atoms, which in its turn can be substituted by hydroxyl or by straight-chain or branched alkoxy having up to 3 carbon atoms,
or
is substituted by the group of the formula -CO-NR⁶R⁷,
wherein
R⁶ and R⁷ have the abovementioned meaning,
R⁵ represents hydrogen, fluorine, chlorine or straight-chain or branched alkyl having up to 4 carbon atoms, or
represents straight-chain or branched perfluoroalkyl having up to 3 carbon atoms, or
represents a group of the formula -OR¹², -NR¹³R¹⁴ or -NR¹³-CO-R¹⁵,
wherein
R¹² denotes hydrogen or straight-chain or branched alkyl having up to 4 carbon atoms,
R¹³ and R¹⁴ are identical or different and have the abovementioned meaning of R⁶ and R⁷,
and
R¹⁵ denotes straight-chain or branched alkyl having up to 6 carbon atoms or phenyl,
A represents piperidyl or pyrrolidinyl which are bonded via the nitrogen atom and are optionally substituted by a radical of the formula
-NR^{9'}R^{10'}, -SO₃H, 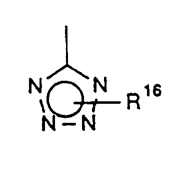 or -CO-R¹⁷,
wherein
R^{9'} and R^{10'} have the abovementioned meaning of R⁹ and R¹⁰ and are identical to or different from these,
R¹⁶ denotes hydrogen, methyl, ethyl or triphenylmethyl
and
R¹⁷ denotes hydroxyl, straight-chain or branched alkoxy having up to 4 carbon atoms, phenoxy or a group of the formula -NR¹⁸R¹⁹,
wherein
R¹⁸ and R¹⁹ are identical or different and denote hydrogen or straight-chain or branched alkyl having up to 3 carbon atoms,
and salts thereof.

4. Sulphonylbenzyl-substituted benzo- and pyridopyridones according to Claim 1 for therapeutic use.

5. Process for the preparation of sulphonylbenzyl-substituted benzo- and pyridopyridones according to Claim 1, characterised in that pyridones of the general formula (II) 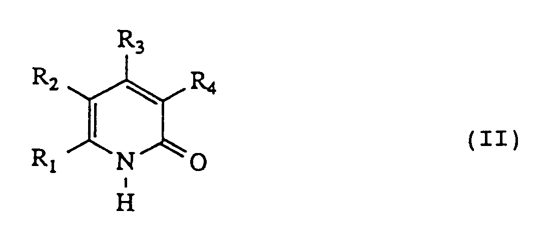 in which
R¹, R², R³ and R⁴ have the abovementioned meaning,
are reacted with compounds of the general formula (III) 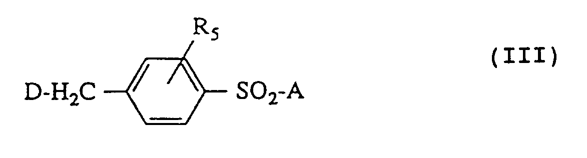 in which
R⁵ and A have the abovementioned meaning
and
D represents halogen, preferably bromine,
in organic solvents and in the presence of a base and if appropriate a catalyst,
and, in the case where R¹⁶ does not represent hydrogen, an alkylation follows,
and, in the case of the acids (R¹⁷= OH), the corresponding esters are hydrolysed,
and, in the case of the esters or amides, an esterification or amidation follows, if appropriate via an activated carboxylic acid stage,
and both the substituents R¹, R² and R⁵ and the substituents of the phenyl and pyridyl ring (R³/R⁴) are varied by customary methods.

6. Medicaments containing sulphonylbenzyl-substituted benzo- and pyridopyridones according to Claim 1.

7. Medicaments according to Claim 6 for the treatment of arterial hypertension and atherosclerosis.

8. Use of sulphonylbenzyl-substituted benzo- and pyridopyridones according to Claim 1 for the preparation of medicaments.

## Claims (Claims for the following Contracting State(s): ES)

1. Process for the preparation of sulphonylbenzyl-substituted benzo- and pyridopyridones of the general formula 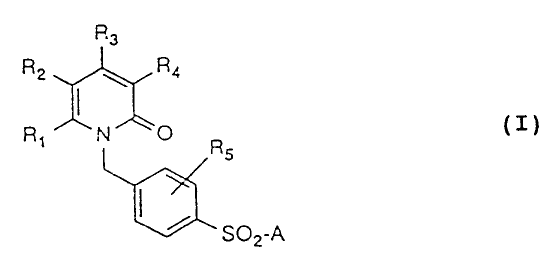 in which
R¹ and R² are identical or different and represent hydrogen or cyano, or
represent straight-chain or branched alkyl, alkenyl or alkinyl having in each case up to 8 carbon atoms, which are optionally substituted by cycloalkyl having 3 to 6 carbon atoms, hydroxyl or by straight-chain or branched alkoxy having up to 6 carbon atoms or phenyl, or
represent cycloalkyl having 3 to 6 carbon atoms, or
represent straight-chain or branched acyl or alkoxycarbonyl having in each case up to 8 carbon atoms, benzyloxycarbonyl or carboxyl, or
represent phenyl, which is optionally substituted up to 3 times in an identical or different manner by halogen, nitro, cyano, hydroxyl, hydroxymethyl, trifluoromethyl or trifluoromethoxy or by straight-chain or branched alkyl or alkoxy having in each case up to 6 carbon atoms, or
represent the group of the formula -CO-NR⁶R⁷, B-R⁸ or -NR⁹R¹⁰,
wherein
R⁶ and R⁷ are identical or different and denote hydrogen, phenyl, straight-chain or branched alkyl having up to 6 carbon atoms or benzyl,
B denotes an oxygen or sulphur atom,
R⁸ denotes straight-chain or branched alkyl having up to 8 carbon atoms,
R⁹ and R¹⁰ are identical or different and have the abovementioned meaning of R⁶ and R⁷ or
R⁹ or R¹⁰ denotes the -SO₂R¹¹ group,
wherein
R¹¹ denotes straight-chain or branched alkyl having up to 6 carbon atoms, benzyl or phenyl, which are optionally substituted by methyl,
R³ and R⁴, including the double bond, form a phenyl or pyridyl ring which is optionally substituted up to 3 times in an identical or different manner by hydroxyl, formyl, carboxyl, halogen, straight-chain or branched acyl or alkoxycarbonyl having in each case up to 8 carbon atoms or straight-chain or branched perfluoroalkyl having up to 6 carbon atoms or by straight-chain or branched alkyl having up to 8 carbon atoms, which in its turn can be substituted by hydroxyl or straight-chain or branched alkoxy having up to 6 carbon atoms, or is substituted by the group of the formula -CONR⁶R⁷,
wherein
R⁶ and R⁷ have the abovementioned meaning,
R⁵ represents hydrogen, nitro, halogen or straight-chain or branched alkyl having up to 8 carbon atoms, or
represents straight-chain or branched perfluoroalkyl having up to 6 carbon atoms, or
represents a group of the formula -OR¹², -NR¹³R¹⁴ or -NR¹³-CO-R¹⁵,
wherein
R¹² denotes hydrogen, straight-chain or branched alkyl having up to 8 carbon atoms or phenyl,
R¹³ and R¹⁴ are identical or different and have the abovementioned meaning of R⁶ and R⁷ and
R¹⁵ denotes straight-chain or branched alkyl having up to 6 carbon atoms or phenyl,
A represents a 3- to 8-membered saturated heterocyclic ring which is bonded via the nitrogen atom and has up to 2 further hetero atoms from the series comprising S, N and O, and is optionally substituted up to twice in an identical or different manner by a radical of the formula
-NR^{9'}R^{10'}, -SO₃H, 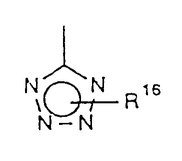 or -CO-R¹⁷,
wherein
R^{9'} and R^{10'} have the abovementioned meaning of R⁹ and R¹⁰ and are identical to or different from these,
R¹⁶ denotes hydrogen, straight-chain or branched alkyl having up to 6 carbon atoms or triphenylmethyl
and
R¹⁷ denotes hydroxyl, straight-chain or branched alkoxy having up to 8 carbon atoms, phenoxy or a group of the formula -NR¹⁸R¹⁹,
wherein
R¹⁸ and R¹⁹ are identical or different and denote hydrogen, straight-chain or branched alkyl having up to 6 carbon atoms or phenyl,
and salts thereof
characterised in that
pyridones of the general formula (II) 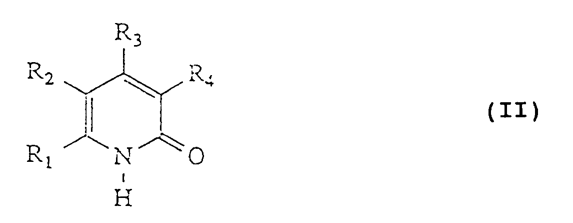 in which
R¹, R², R³ and R⁴ have the abovementioned meaning,
are reacted with compounds of the general formula (III) 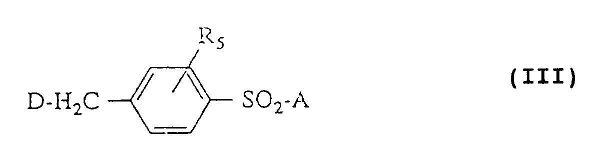 in which
R⁵ and A have the abovementioned meaning
and
D represents halogen, preferably bromine,
in organic solvents and in the presence of a base and if appropriate a catalyst,
and, in the case where R¹⁶ does not represent hydrogen, an alkylation follows,
and, in the case of the acids (R¹⁷ = OH), the corresponding esters are hydrolysed,
and, in the case of the esters or amides, an esterification or amidation follows, if appropriate via an activated carboxylic acid stage,
and both the substituents R¹, R² and R⁵ and the substituents of the phenyl and pyridyl ring (R³/R⁴) are varied by customary methods.

## Revendications (Revendications pour l'(les) Etat(s) contractant(s) suivant(s): AT, BE, CH, DE, DK, FR, GB, GR, IE, IT, LI, LU, MC, NL, PT, SE)

1. Benzo- et pyridopyridones à substituant sulfonylbenzyle, de formule générale 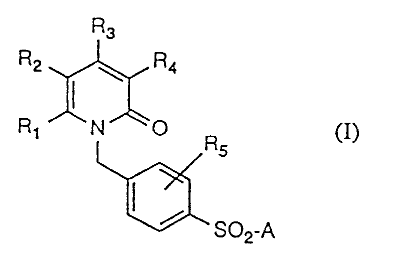 dans laquelle
R¹ et R² sont identiques ou différents et représentent de l'hydrogène, un groupe cyano ou un groupe alkyle, un groupe alcényle ou un groupe alcynyle linéaire ou ramifié ayant chacun jusq'à 8 atomes de carbone et chacun pouvant être substitué par un radical cycloalkyle ayant 3 à 6 atomes de carbone, un radical hydroxy, un radical alkoxy linéaire ou ramifié ayant jusqu'à 6 atomes de carbone ou un radical phényle, ou
un groupe cycloalkyle ayant 3 à 6 atomes de carbone,
un groupe acyle ou alkoxycarbonyle linéaire ou ramifié ayant chacun jusqu'à 8 atomes de carbone, un groupe benzyloxycarbonyle ou carboxy, ou bien
un groupe phényle gui est substitué le cas échéant jusqu'à 3 fois, identiques ou différentes, par un radical halogéno, nitro, cyano, hydroxy, hydroxyméthyle, trifluorométhyle, trifluorométhoxy ou par un radical alkyle ou alkoxy linéaire ou ramifié ayant chacun jusqu'à 6 atomes de carbone, ou bien
le groupe de formule -CO-NR⁶R⁷, B-R⁸ ou -NR⁹R¹⁰, dans laquelle
R⁶ et R⁷ sont identiques ou différents et représentent de l'hydrogène, un groupe phényle, un groupe alkyle linéaire ou ramifié ayant jusqu'à 6 atomes de carbone ou un groupe benzyle,
B est un atome d'oxygène ou de soufre,
R⁸ est un groupe alkyle linéaire ou ramifié ayant jusqu'à 8 atomes de carbone,
R⁹ et R¹⁰ sont identiques ou différents et ont la définition indiquée ci-dessus pour R⁶ et R⁷, ou bien
R⁹ ou R¹⁰ représente le groupe -SO₂R¹¹,
dans lequel
R¹¹ est un radical alkyle linéaire ou ramifié ayant jusqu'à 6 atomes de carbone, benzyle ou phényle, ces radicaux étant éventuellement substitués par un groupe méthyle,
R³ et R⁴ forment conjointement avec la double liaison un noyau phényle ou pyridyle qui est substitué le cas échéant jusqu'à 3 fois, identiques ou différentes, par un radical hydroxy, formyle, carboxy, halogéno, acyle ou alkoxycarbonyle linéaire ou ramifié ayant chacun jusqu'à 8 atomes de carbone, perfluoralkyle linéaire ou ramifié ayant jusqu'à 6 atomes de carbone ou alkyle linéaire ou ramifié ayant jusqu'à 8 atomes de carbone et pouvant porter quant à lui un substituant hydroxy ou alkoxy linéaire ou ramifié ayant jusqu'à 6 atomes de carbone, ou par le groupe de formule -CONR⁶R⁷,
dans laquelle
R⁶ et R⁷ ont la définition indiquée ci-dessus,
R⁵ est de l'hydrogène, un groupe nitro, halogéno, alkyle linéaire ou ramifié ayant jusqu'à 8 atomes de carbone, ou bien
un groupe perfluoralkyle linéaire ou ramifié ayant jusqu'à 6 atomes de carbone, ou bien
un groupe de formule -OR¹², -NR¹³R¹⁴ ou -NR¹³-CO-R¹⁵,
dans laquelle
R¹² est de l'hydrogène, un radical alkyle linéaire ou ramifié ayant jusqu'à 8 atomes de carbone ou un radical phényle,
R¹³ et R¹⁴ sont identiques ou différents et ont la définition indiquée ci-dessus pour R⁶ et R⁷,
et
R¹⁵ est un groupe alkyle linéaire ou ramifié ayant jusqu'à 6 atomes de carbone ou un groupe phényle,
A représente un hétérocycle saturé de 3 à 8 chaînons, lié par l'intermédiaire de l'atome d'azote, ayant jusqu'à 2 autres hétéroatomes de la série S, N ou O et éventuellement substitué jusqu'à 2 fois identiques ou différentes par un reste de formule
-NR^{9'}R^{10'}, -SO₃H , 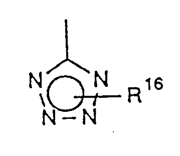 ou -CO-R¹⁷
dans laquelle
R^{9'} et R^{10'} ont la définition indiquée ci-dessus pour R⁹ et R¹⁰ et y sont identiques ou en sont différents,
R¹⁶ représente de l'hydrogène, un groupe alkyle linéaire ou ramifié ayant jusqu'à 6 atomes de carbone ou un groupe triphénylméthyle
et
R¹⁷ est un groupe hydroxy, alkoxy linéaire ou ramifié ayant jusqu'à 8 atomes de carbone, un groupe phénoxy ou un groupe de formule -NR¹⁸R¹⁹,
dans laquelle
R¹⁸ et R¹⁹ sont identiques ou différents et représentent de l'hydrogène ou un groupe alkyle linéaire ou ramifié ayant jusqu'à 6 atomes de carbone ou un groupe phényle,
et leurs sels.

2. Benzo- et pyridopyridones à substituant sulfonylbenzyle suivant la revendication 1,
dans lesquelles
R¹ et R² sont identiques ou différents et représentent de l'hydrogène, un groupe cyano ou un groupe alkyle, un groupe alcényle ou un groupe alcynyle linéaire ou ramifié ayant chacun jusqu'à 6 atomes de carbone, ces groupes étant éventuellement substitués par un radical cyclopropyle, cyclopentyle, cyclohexyle, hydroxy ou par un radical alkoxy linéaire ou ramifié ayant jusqu'à 4 atomes de carbone ou un radical phényle, ou bien
un groupe cyclopropyle, cyclopentyle ou cyclohexyle,
un groupe acyle ou alkoxycarbonyle linéaire ou ramifié ayant chacun jusqu'à 6 atomes de carbone, benzyloxycarbonyle ou carboxy, ou bien un groupe phényle qui est substitué le cas échéant jusqu'à 2 fois, identiques ou différentes, par du fluor, du chlore, du brome, un radical trifluorométhyle, trifluorométhoxy, hydroxyméthyle ou par un radical alkyle ou alkoxy linéaire ou ramifié ayant chacun jusqu'à 4 atomes de carbone, ou bien
un groupe de formule -CO-NR⁶R⁷, B-R⁸ ou -NR⁹R¹⁰, dans laquelle
R⁶ et R⁷ sont identiques ou différents et représentent de l'hydrogène, un radical phényle, alkyle linéaire ou ramifié ayant jusqu'à 4 atomes de carbone, ou benzyle,
B est un atome d'oxygène ou de soufre,
R⁸ est un radical alkyle linéaire ou ramifié ayant jusqu'à 6 atomes de carbone,
R⁹ et R¹⁰ sont identiques ou différents et ont la définition indiquée ci-dessus pour R⁶ et R⁷, ou bien R⁹ ou R¹⁰ représente le groupe -SO₂R¹¹-,
dans lequel
R¹¹ est un radical alkyle linéaire ou ramifié ayant jusqu'à 4 atomes de carbone, phényle ou tolyle,
R³ et R⁴ forment, conjointement avec la double liaison, un noyau phényle ou pyridyle qui est substitué le cas échéant jusqu'à deux fois, identiques ou différentes, par un radical hydroxy, formyle, carboxy, fluoro, chloro, bromo, un radical acyle ou alkoxycarbonyle linéaire ou ramifié ayant chacun jusqu'à 6 atomes de carbone, un radical perfluoralkyle linéaire ou ramifié ayant jusqu'à 4 atomes de carbone ou un radical alkyle linéaire ou ramifié ayant jusqu'à 6 atomes de carbone, pouvant porter quant à lui un substituant hydroxy ou un substituant alkoxy linéaire ou ramifié ayant jusqu'à 4 atomes de carbone, ou par le groupe de formule -CONR⁶R⁷,
dans laquelle
R⁶ et R⁷ ont la définition indiquée ci-dessus,
R⁵ est de l'hydrogène, un radical nitro, fluoro, chloro, bromo, un radical alkyle linéaire ou ramifié ayant jusqu'à 6 atomes de carbone ou un radical perfluoralkyle linéaire ou ramifié ayant jusqu'à 4 atomes de carbone, ou bien un groupe de formule -OR¹², -NR¹³R¹⁴ ou -NR¹³-CO-R¹⁵,
dans laquelle
R¹² est de l'hydrogène ou un radical alkyle linéaire ou ramifié ayant jusqu'à 6 atomes de carbone,
R¹³ et R¹⁴ sont identiques ou différents et ont la définition indiquée ci-dessus pour R⁶ et R⁷
et
R¹⁵ est un radical alkyle linéaire ou ramifié ayant jusqu'à 6 atomes de carbone ou un radical phényle
A est un groupe pipéridyle, pyrrolidinyle ou morpholinyle lié par l'intermédiaire de l'atome d'azote, ces groupes étant éventuellement substitués par un reste de formule
-NR^{9'}R^{10'}, -SO₃H , 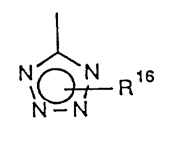 ou -CO-R¹⁷
dans laquelle
R^{9'} et R^{10'} ont la définition indiquée ci-dessus pour R⁹ et R¹⁰ et y sont identiques ou en sont différents,
R¹⁶ représente de l'hydrogène, un groupe alkyle linéaire ou ramifié ayant jusqu'à 4 atomes de carbone ou un groupe triphénylméthyle
et
R¹⁷ est un groupe hydroxy, alkoxy linéaire ou ramifié ayant jusqu'à 6 atomes de carbone, phénoxy ou un groupe de formule -NR¹⁸R¹⁹,
dans laquelle
R¹⁸ et R¹⁹ sont identiques ou différents et représentent de l'hydrogène ou un radical alkyle linéaire ou ramifié ayant jusqu'à 4 atomes de carbone,
et leurs sels.

3. Benzo- et pyridopyridones à substituant sulfonylbenzyle suivant la revendication 1, dans lesquelles
R¹ et R² sont identiques ou différents et représentent de l'hydrogène, un groupe cyano ou un groupe alkyle linéaire ou ramifié ayant jusqu'à 4 atomes de carbone, qui est substitué le cas échéant par un radical cyclopropyle,
ou bien
un groupe cyclopropyle ou phényle, ou bien
un groupe acyle ou alkoxycarbonyle linéaire ou ramifié ayant chacun jusqu'à 4 atomes de carbone, benzyloxycarbonyle ou carboxy, ou bien un groupe de formule -CO-NR⁶R⁷, B-R⁸ ou -NR⁹R¹⁰,
dans laquelle
R⁶ et R⁷ sont identiques ou différents et représentent de l'hydrogène, un groupe phényle, éthyle ou benzyle,
B est un atome d'oxygène ou de soufre,
R⁸ est un groupe alkyle linéaire ou ramifié ayant jusqu'à 4 atomes de carbone,
R⁹ et R¹⁰ sont identiques ou différents et ont la définition indiquée ci-dessus pour R⁶ et R⁷, ou bien
R⁹ ou R¹⁰ représentent le groupe -SO₂R¹¹,
dans lequel
R¹¹ est un radical méthyle, phényle ou tolyle,
R³ et R⁴ forment, conjointement avec la double liaison, un noyau phényle ou pyridyle condensé qui est substitué le cas échéant jusqu'à 2 fois, identiques ou différentes, par un radical hydroxy, carboxy, fluoro, chloro, un radical acyle ou alkoxycarbonyle linéaire ou ramifié ayant chacun jusqu'à 4 atomes de carbone ou un radical perfluoralkyle linéaire ou ramifié ayant jusqu'à 3 atomes de carbone ou un radical alkyle linéaire ou ramifié ayant jusqu'à 4 atomes de carbone, pouvant porter quant à lui un radical hydroxy ou un radical alkoxy linéaire ou ramifié ayant jusqu'à 3 atomes de carbone, ou bien
par le groupe de formule -CO-NR⁶R⁷,
dans laquelle
R⁶ et R⁷ ont la définition indiquée ci-dessus,
R⁵ est de l'hydrogène, du fluor, du chlore, un radical alkyle linéaire ou ramifié ayant jusqu'à 4 atomes de carbone, ou bien
un groupe perfluoralkyle linéaire ou ramifié ayant jusqu'à 3 atomes de carbone, ou bien
un groupe de formule -OR¹², -NR¹³R¹⁴ ou -NR¹³-CO-R¹⁵,
dans laquelle
R¹² est de l'hydrogène ou un radical alkyle linéaire ou ramifié ayant jusqu'à 4 atomes de carbone,
R¹³ et R¹⁴ sont identiques ou différents et ont la définition indiquée ci-dessus pour R⁶ et R⁷
et
R¹⁵ est un groupe alkyle linéaire ou ramifié ayant jusqu'à 6 atomes de carbone ou un groupe phényle
A est un noyau pipéridyle ou pyrrolidinyle lié par l'intermédiaire de l'atome d'azote, ces groupes étant substitués le cas échéant par un reste de formule
-NR^{9'}R^{10'}, -SO₃H , 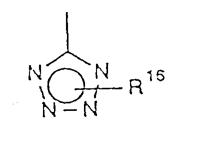 ou -CO-R¹⁷
dans laquelle
R^{9'} et R^{10'} ont la définition indiquée ci-dessus pour R⁹ et R¹⁰ et y sont identiques ou en sont différents,
R¹⁶ est de l'hydrogène, un groupe méthyle, éthyle ou triphénylméthyle,
et
R¹⁷ est un groupe hydroxy, alkoxy linéaire ou ramifié ayant jusqu'à 4 atomes de carbone, phénoxy ou un groupe de formule -NR¹⁸R¹⁹,
dans laquelle
R¹⁸ et R¹⁹ sont identiques ou différents et représentent de l'hydrogène ou un radical alkyle linéaire ou ramifié ayant jusqu'à 3 atomes de carbone,
et leurs sels.

4. Benzo- et pyridopyridones à substituant sulfonylbenzyle suivant la revendication 1, destinées à une application thérapeutique.

5. Procédé de production de benzo- et pyridopyridones à substituant sulfonylbenzyle suivant la revendication 1, caractérisé en ce qu'on fait réagir des pyridones de formule générale (II) 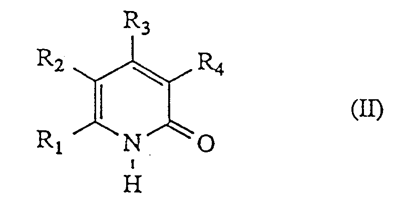 dans laquelle
R¹, R², R³ et R⁴ ont la définition indiquée ci-dessus, avec des composés de formule générale (III) 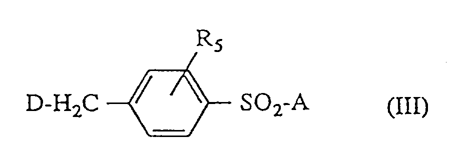
dans laquelle
R⁵ et A ont la définition indiquée ci-dessus
et
D est un halogène, de préférence le brome,
dans des solvants organiques et en présence d'une base et, le cas échéant, d'un catalyseur,
et au cas où R¹⁶ ne représente pas de l'hydrogène, on effectue ensuite une alkylation,
dans le cas des acides (R¹⁷=OH), on saponifie les esters correspondants,
dans le cas des esters ou des amides, on effectue ensuite le cas échéant une estérification ou respectivement une amidation en passant éventuellement par un stade d'acide carboxylique activé,
en faisant varier tant les substituants R¹, R² et R⁵ que les substituants du noyau phényle et du noyau pyridyle (R³/R⁴) par des procédés classiques.

6. Médicament contenant des benzo- et pyridopyridones à substituant sulfonylbenzyle suivant la revendication 1.

7. Médicament suivant la revendication 6, destiné au traitement de l'hypertonie artérielle et de l'athérosclérose.

8. Utilisation de benzo- et pyridopyridones à substituant sulfonylbenzyle suivant la revendication 1 pour la préparation de médicaments.

## Revendications (Revendications pour l'(les) Etat(s) contractant(s) suivant(s): ES)

1. Procédé de production de benzo- et pyridopyridones à substituant sulfonylbenzyle de formule générale 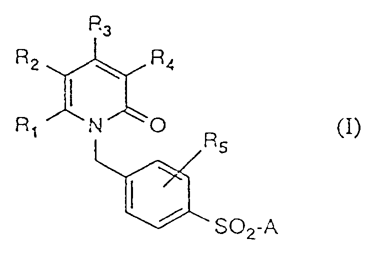 dans laquelle
R¹ et R² sont identiques ou différents et représentent de l'hydrogène, un groupe cyano ou un groupe alkyle, un groupe alcényle ou un groupe alcynyle linéaire ou ramifié ayant chacun jusqu'à 8 atomes de carbone et chacun pouvant être substitué par un radical cycloalkyle ayant 3 à 6 atomes de carbone, un radical hydroxy, un radical alkoxy linéaire ou ramifié ayant jusqu'à 6 atomes de carbone ou un radical phényle, ou
un groupe cycloalkyle ayant 3 à 6 atomes de carbone,
un groupe acyle ou alkoxycarbonyle linéaire ou ramifié ayant chacun jusqu'à 8 atomes de carbone, un groupe benzyloxycarbonyle ou carboxy, ou bien
un groupe phényle qui est substitué le cas échéant jusqu'à 3 fois, identiques ou différentes, par un radical halogéno, nitro, cyano, hydroxy, hydroxyméthyle, trifluorométhyle, trifluorométhoxy ou par un radical alkyle ou alkoxy linéaire ou ramifié ayant chacun jusqu'à 6 atomes de carbone, ou bien
le groupe de formule -CO-NR⁶R⁷, B-R⁸ ou -NR⁹R¹⁰,
dans laquelle
R⁶ et R⁷ sont identiques ou différents et représentent de l'hydrogène, un groupe phényle, un groupe alkyle linéaire ou ramifié ayant jusqu'à 6 atomes de carbone ou un groupe benzyle,
B est un atome d'oxygène ou de soufre,
R⁸ est un groupe alkyle linéaire ou ramifié ayant jusqu'à 8 atomes de carbone,
R⁹ et R¹⁰ sont identiques ou différents et ont la définition indiquée ci-dessus pour R⁶ et R⁷, ou bien
R⁹ ou R¹⁰ représente le groupe -SO₂R¹¹,
dans lequel
R¹¹ est un radical alkyle linéaire ou ramifié ayant jusqu'à 6 atomes de carbone, benzyle ou phényle, ces radicaux étant éventuellement substitués par un groupe méthyle,
R³ et R⁴ forment conjointement avec la double liaison un noyau phényle ou pyridyle qui est substitué le cas échéant jusqu'à 3 fois, identiques ou différentes, par un radical hydroxy, formyle, carboxy, halogéno, acyle ou alkoxycarbonyle linéaire ou ramifié ayant chacun jusqu'à 8 atomes de carbone, perfluoralkyle linéaire ou ramifié ayant jusqu'à 6 atomes de carbone ou alkyle linéaire ou ramifié ayant jusqu'à 8 atomes de carbone et pouvant porter quant à lui un substituant hydroxy ou alkoxy linéaire ou ramifié ayant jusqu'à 6 atomes de carbone, ou par le groupe de formule -CONR⁶R⁷,
dans laquelle
R⁶ et R⁷ ont la définition indiquée ci-dessus,
R⁵ est de l'hydrogène, un groupe nitro, halogéno, alkyle linéaire ou ramifié ayant jusqu'à 8 atomes de carbone, ou bien
un groupe perfluoralkyle linéaire ou ramifié ayant jusqu'à 6 atomes de carbone, ou bien
un groupe de formule -OR¹², -NR¹³R¹⁴ ou -NR¹³-CO-R¹⁵,
dans laquelle
R¹² est de l'hydrogène, un radical alkyle linéaire ou ramifié ayant jusqu'à 8 atomes de carbone ou un radical phényle,
R¹³ et R¹⁴ sont identiques ou différents et ont la définition indiquée ci-dessus pour R⁶ et R⁷,
et
R¹⁵ est un groupe alkyle linéaire ou ramifié ayant jusqu'à 6 atomes de carbone ou un groupe phényle,
A représente un hétérocycle saturé de 3 à 8 chaînons, lié par l'intermédiaire de l'atome d'azote, ayant jusqu'à 2 autres hétéroatomes de la série S, N ou O et éventuellement substitué jusqu'à 2 fois identiques ou différentes par un reste de formule
-NR^{9'}R^{10'}, -SO₃H , 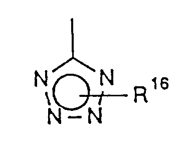 ou -CO-R¹⁷
dans laquelle
R^{9'} et R^{10'} ont la définition indiquée ci-dessus pour R⁹ et R¹⁰ et y sont identiques ou en sont différents,
R¹⁶ représente de l'hydrogène, un groupe alkyle linéaire ou ramifié ayant jusqu'à 6 atomes de carbone ou un groupe triphénylméthyle
et
R¹⁷ est un groupe hydroxy, alkoxy linéaire ou ramifie ayant jusqu'à 8 atomes de carbone, un groupe phénoxy ou un groupe de formule -NR¹⁸R¹⁹,
dans laquelle
R¹⁸ et R¹⁹ sont identiques ou différents et représentent de l'hydrogène ou un groupe alkyle linéaire ou ramifié ayant jusqu'à 6 atomes de carbone ou un groupe phényle,
et de leurs sels
caractérisé en ce qu'on fait réagir des pyridones de formule générale (II) 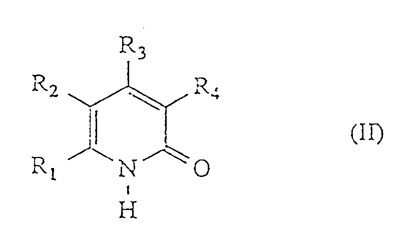 dans laquelle
R¹, R², R³ et R⁴ ont la définition indiquée ci-dessus, avec des composés de formule générale (III) 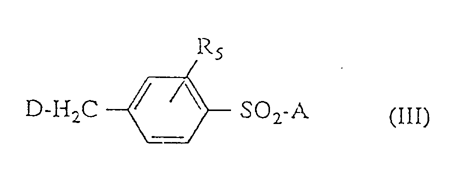
dans laquelle
R⁵ et A ont la définition indiquée ci-dessus
et
D est un halogène, de préférence le brome,
dans des solvants organiques et en présence d'une base et, le cas échéant, d'un catalyseur,
et au cas où R¹⁶ ne représente pas de l'hydrogène, on effectue ensuite une alkylation,
dans le cas des acides (R¹⁷=OH), on saponifie les esters correspondants,
dans le cas des esters ou des amides, on effectue ensuite le cas échéant une estérification ou respectivement une amidation en passant éventuellement par un stade d'acide carboxylique activé,
en faisant varier tant les substituants R¹, R² et R⁵ que les substituants du noyau phényle et du noyau pyridyle (R³/R⁴) par des procédés classiques.
